# EUROPEAN PATENT SPECIFICATION

(11) **EP 2 229 150 B1**
(45) Date of publication and mention of the grant of the patent: **04.07.2012**
(21) Application number: 08857500.6
(22) Date of filing: 03.12.2008
(51) Int. Cl.: A61K 9/16, A61K 31/196, A61K 31/519, A61P 25/18, A61P 29/00

(54) **SYSTEMS AND METHODS FOR DELIVERY OF MATERIALS**
SYSTEME UND VERFAHREN ZUR ABGABE VON MATERIALIEN
SYSTÈMES ET PROCÉDÉS POUR DÉLIVRER DES SUBSTANCES

(30) Priority: 04.12.2007 US 5400 P; 04.12.2007 US 999415; 04.06.2008 US 131123 P; 10.06.2008 US 131716 P
(43) Date of publication of application: 22.09.2010
(60) Divisional application: 12169129.9
(73) Proprietor: LANDEC CORPORATION, Menlo Park California 94025 (US)
(72) Inventor: TAFT, David, D., Menlo Park CA 94025 (US); BITLER, Steven, P., Menlo Park CA 9405 (US); ZHENG, Qiang, Menlo Park CA 94025 (US); TZANNIS, Stelios, T., Menlo CA 94025 (US); BELL, Adam, W, CA 94127 (US); DAI, Wei-guo, Sunnyvale CA 94086 (US); OTTENSMANN, Sandra, N, Mountain View CA 94040 (US); BALACHANDER, Natarajan, West Lafayette IN 47906 (US)
(74) Representative: Brewster, Andrea Ruth
(86) International application number: PCT/US2008/013335
(87) International publication number: WO 2009/073192

(56) References cited:
- WO-A-01/19333
- WO-A-98/11166
- WO-A-2008/066657
- WO-A-2008/070118
- DE-A1- 19 908 753
- US-A- 5 736 125

## Description

### FIELD OF THE INVENTION

This invention relates to pharmaceutical formulations and their preparation.

### BACKGROUND

There are many known polymeric systems for the delivery of drugs. A continuing problem is obtaining a desired loading and delivery profile at a desired location and at a desired time. WO 01/19333 discloses cosmetic and pharmaceutical formulations containing an oil which is thickened by a side chain crystalline (SCC) polymer which has a heat of fusion of at least 20 J/g and a melting point above 0°C. The backbone of the SCC polymer contains multiple repeating units containing long chain n-alkyl moieties, and may also contain other repeating units, for example units derived from monomers containing desired functional groups, including for example N-vinyl pyrrolidinone, N,N-dimethylaminoethylacrylate and 2-hydroxyethyl acrylate. DE 19908753 discloses biodegradable pharmaceutical compositions comprising a drug and a mixture of polymers which are polymerization products of hydroxy carboxylic acids, preferably lactic acid and glycolic acid; the polymers are not crystalline.

### SUMMARY OF THE INVENTION

This invention provides pharmaceutical formulations which comprise an ECC polymer and a drug associated with the ECC polymer, the ECC polymer
(A) comprising a plurality of polymeric molecules each of which consists essentially of
   (i) a polymer backbone which comprises a plurality of repeating units having the formula

      -CF¹F²-CO-O- (1)

      wherein
      F¹ is hydrogen and F² is hydrogen or methyl, the repeating units being the same or different, and
   (ii) at least one terminal unit which has the formula

      - b- Cy (2)

      wherein
      Cy is an n-alkyl moiety containing 18-24 carbon atoms, and
      b is a bond or a moiety which has a valence of at least 2, which links the Cy moiety to the polymer backbone, and which optionally contains one or more additional Cy moieties;
(B) having a crystalline melting temperature, Tp, of at least 40°C, an onset of melting temperature, To, such that the value of (Tp -To) is less than Tp^{0.7}, and a heat of fusion, ΔH, of at least 5 J/g, Tp, To and ΔH being measured on a differential scanning calorimeter (DSC) as hereinafter described; and
(C) having a number average molecular weight, Mn, measured as hereinafter described, of less than 10,000.

The invention also provides a method of making a pharmaceutical formulation as defined above by mixing a drug with an ECC polymer as defined above, the drug having a maximum temperature to which it can be exposed without damage, and the drug being mixed with the polymer at a temperature at which the polymer is liquid and which is below the maximum temperature.

The term "CYSC polymer" is used herein to denote a polymer which:
(A) comprises polymeric molecules having a backbone and comprising at least one moiety which
   (i) has the formula -b-Cy, and
   (ii) either
      (A) forms part of a repeating unit of the backbone, the repeating unit having formula (1) below 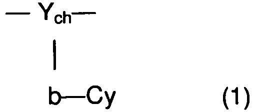 where Y_{ch} is a moiety forming part of the backbone, b is a bond or moiety which links the Cy moiety to Y_{ch}, and Cy is a moiety which is associated with other moieties (which may also be Cy moieties) to provide the CYSC polymer with crystallinity;
         or
      (B) forms part of a terminal unit of the backbone, the terminal unit having formula (2) below

         -Yₜₑᵣₘ - b-Cy (2)

         where Yₜₑᵣₘ is a moiety at the end of the backbone, and b and Cy are as defined in formula (1);
   at least a majority by weight, preferably at least 90% by weight, particularly substantially all, of the Cy moieties being present in repeating units of formula (1), and
(B) has a crystalline melting temperature, Tp, of at least 0°C and a heat of fusion, Δh, of at least 3 J/g which result from association of the Cy moieties, Tp and ΔH being measured on a differential scanning calorimeter (DSC) as hereinafter described.

In many CYSC polymers, the backbone of the polymeric molecules comprises repeating units having formula (3) below 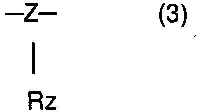 where Z is a moiety forming part of the backbone and Rz represents a moiety which does not comprise a Cy moiety. Many useful CYSC polymers have an amphiphilic character, with the Cy moieties providing hydrophobic characteristics and the Z(Rz) moieties providing hydrophilic characteristics.

### BRIEF DESCRIPTION OF THE DRAWINGS

The invention is illustrated in the accompanying drawings in which the Figures summarize results obtained in the Examples, as further described below. In each of the Figures, unless otherwise noted, the vertical axis shows the total percentage release ("cumulative release") of the release material, the horizontal axis shows the time in days, and the different curves are identified by reference to the polymer or sample ID #marked on the Figure.

### DETAILED DESCRIPTION OF THE INVENTION

In this specification: --
(1) Reference is made to particular features of the invention (including for example components, ingredients, elements, devices, apparatus, systems, groups, ranges, method steps, test results, etc). It is to be understood that the disclosure of the invention in this specification includes all possible combinations of such particular features. For example, where a particular feature is disclosed in the context of a particular embodiment or claim, that feature can also be used, to the extent appropriate, in the context of other particular embodiments and claims, and in the invention generally.
(2) The singular forms "a", "an", and "the" include plural reference unless the context clearly dictates otherwise. Thus, for example, a reference to "a part" includes a plurality of such parts.
(3) The term "comprises" and grammatical equivalents thereof are used to mean that, in addition to the features specifically identified, other features are optionally present. For example a formulation which comprises an ECC polymer and a drug can contain a single ECC polymer and a single drug, or two or more ECC polymers and/or two or more drugs, and optionally contains one or more other ingredients which are not ECC polymers, for example other ingredients as disclosed herein.
(4) The term "consisting essentially of" and grammatical equivalents thereof are used to mean that, in addition to the features specifically identified, other features may be present which do not materially alter the disclosed and/or claimed invention.
(5) The term "at least" followed by a number is used to denote the start of a range beginning with that number (which may be a range having an upper limit or no upper limit, depending on the variable being defined). For example "at least 1" means 1 or more than 1, and "at least 80%" means 80% or more than 80%.
(6) The term "at most" followed by a number is used to denote the end of a range ending with that number (which may be a range having 1 or 0 as its lower limit, or a range having no lower limit, depending upon the variable being defined). For example, "at most 4" means 4 or less than 4, and "at most 40%" means 40% or less than 40 %.
(7) A range written as " (a first number) to (a second number)" or "(a first number) - (a second number)" means a range whose lower limit is the first number and whose upper limit is the second number. For example, "from 8 to 20 carbon atoms" or "8-20 carbon atoms" means a range whose lower limit is 8 carbon atoms, and whose upper limit is 20 carbon atoms.
(8) The terms "plural", "multiple", "plurality" and "multiplicity" are used herein to denote two or more than two features.
(9) When a method is described as comprising two or more defined steps, the defined steps can be carried out in any order or simultaneously (except where the context excludes that possibility), and the method can optionally include one or more other steps which are carried out before any of the defined steps, between two of the defined steps, or after all the defined steps (except where the context excludes that possibility).
(10) When reference is made to two or more features, this includes the possibility that the two or more features are replaced by a lesser number or greater number of features providing the same function (except where the context excludes that possibility).
(11) The numbers given should be construed with the latitude appropriate to their context and expression; for example, each number is subject to variation which depends on the accuracy with which it can be measured by methods conventionally used by those skilled in the art.
(12) Parts, ratios and percentages are by weight, except where otherwise noted.
(13) Temperatures are in degrees Centigrade (°C).
(14) Molecular weights of polymers are in Daltons; are number average molecular weights (Mn) unless stated to be weight average molecular weights (Mw); and are measured by gel permeation chromatography (GPC) with a light scattering detection method, for example using a DAWN DSP laser photometer from Wyatt Technology.
(15) The terms "melting point" (often abbreviated to Tp), "onset of melting temperature" (often abbreviated to To) and "heat of fusion" (which is a measure of crystallinity of the polymer, is expressed in J/g and is often abbreviated to ΔH) are well known to polymer technologists and refer to quantities determined using a differential scanning calorimeter (hereinafter DSC), e.g. a Q 100 DSC from TA Instruments, at a rate of temperature change of 10ºC/min, e.g. from - 10 to 150°C. Tp is the peak melting temperature, To is the temperature at the intersection of the baseline of the DSC peak and the onset line, the onset line being defined as the tangent to the steepest part of the DSC curve below Tp, and ΔH is the heat of fusion associated with the endotherm or exotherm as calculated by the DSC and is reported in J/g. Unless otherwise stated, the values of Tp, To and ΔH are measured on the second heat cycle.
(16) Bulk viscosities are given in centipoise and are measured using a Brookfield LVT viscometer with an electronically thermostat controlled thermal heater, controlled for example to 95°C, and small sample adapter using spindles 4 and 7.
(17) Solubility parameters are calculated using the method described in D.W. van Krevelen, "Properties of Polymers" Elsevier, 1997, p. 200-214 especially p. 214 and reported in J^{1/2}/cm ^{3/2}.
(18) The term "associated" and grammatical variations thereof include any type of interaction, including chemical bonds (for example, covalent, ionic and hydrogen bonds) and/or Van der Waals forces, and/or polar and non-polar interactions through other physical constraints provided by molecular structure, and interactions through physical mixing.
(19) The term "pharmaceutical formulation" means a composition which (i) is suitable for administration to a human being or other mammal or which can be treated, e.g. sterilized, to make it suitable for such administration, and (ii) comprises at least one drug and at least one ECC polymer. The term "drug" means a material which is biologically active in a human being or other mammal, locally and/or systemically.
(20) The term "therapeutically effective amount" or "therapeutically effective dosage" means an amount of a drug which results in a desired therapeutic effect.
(21) The term "organism" includes human beings and other mammals, and living tissue which is not part of a mammal.
(22) The term "therapeutically effective amount" means an amount of a drug which produces a desired therapeutic effect.
(23) The term "diagnostic agent" means any chemical moiety that can be used for diagnosis or in a diagnostic test. For example, diagnostic agents include imaging agents containing radioisotopes, contrasting agents containing for example iodine, enzymes, fluorescent substances and the like.
(24) The term "treatment" means administration of a composition to a site, e.g the administration of a pharmaceutical formulation to an individual in order to alter a physiological state, whether or not the process includes a curative element.
(25) "Controlled release" of a drug means release of the material in a predetermined or adjustable way such that the amount or rate or timing of release is pre-set or is altered in a desired way.
(26) The terms "controlled release device", "controlled release dosage form" and similar terms mean any formulation or device wherein the release rate (e.g., rate of timing of release) of a drug or other desired substance contained therein is controlled by the device or dosage form itself and/or by the environment of use. Controlled drug delivery includes delivery of an amount of drug to a particular target site at a particular time, for example delivery of a bolus of drug to a tumor site.
(27) "Sustained release" of a drug or other material means release over an extended period of time, for example minutes, hours or days, such that less than all the bioactive material is released initially. A sustained release rate may provide, for example, a release of a specified amount of a drug from a pharmaceutical formulation, over a certain time period, under physiological conditions or in an in vitro test.
(28) "Bolus" release means release of a large dose, for example substantially all of a drug at one time or over a short period of time. Bolus release can be preceded or followed by sustained release.
(29) The term "burst effect" is used, often in the context of drug delivery, to mean release of a bioactive material from a composition in an amount or at a rate which is higher than is desired (typically, in drug delivery, higher than the therapeutic window). A burst is generally followed by a rapidly decreasing rate of release. A burst effect may be defined as the release of more than a defined threshold proportion of the bioactive material over a defined time under defined conditions. The defined conditions can for example be physiological conditions (e.g. gastrointestinally for a pill or subcutaneously for an implant) or the conditions in a suitable in vitro test (for example in phosphate-buffered saline at about 20°C or about 37°C, or a test as described in the Examples below). The threshold proportion can for example be 20%, 30%, 40%, 50%, 60%, 70%, 80%, 90%, or 95%, and the defined time can for example be 30 minutes, 1 hour, 2 hours, 3 hours, 6 hours, 8 hours, 10 hours, 12 hours, 16 hours, 20 hours, 24 hours, 36 hours, or 48 hours. For example, a pharmaceutical formulation which does not display a significant burst effect may be one which releases less than 10% of the drug over a 3 hour period after administration, or over a 24-hour period after administration.
(30) The term "alkyl" includes alkyl moieties which are straight chain alkyl moieties, branched chain alkyl moieties, cycloalkyl moieties, and moieties which consist essentially of two or more of straight chain alkyl, branched chain alkyl and cycloalkyl moieties.
(31) The term "bioerodable" (sometimes alternatively "biodegradable") as applied to an ECC polymer or to a composition means that the polymer or composition, when placed in the human body, is eliminated from the human body, the polymer being eliminated without change or as one or more lower molecular weight products resulting from the degradation of the polymer in the human body. The elimination may take place relatively rapidly, e.g. in a period of up to 10 weeks, but is more often longer, for example over a period of up to 1 year or longer, e.g. up to 3 years.
(32) Some of the structural formulas given below for ECC polymers show the repeating units in the general form

   -(-unit#1-)ₓ-(- unit#1-)_{y}-

This representation is used to denote polymers in which the different repeating units are distributed randomly and/or are distributed in blocks containing only one of the repeating units. Thus, the polymers represented by these formulas can be either random copolymers or block copolymers.

The tables below sets out other abbreviations used in this specification, and the meanings to be attributed to them.

| Abbreviation | Meaning |
|---|---|
| Cx | a linear moiety containing x carbon atoms |
| CxAcid | An acid of formula Cx-1.COOH, e.g.C22Acid is behenic acid. |
| CxOH | An alcohol of formula CxOH, e.g. C22OH is behenyl alcohol. |
| CxA | an n-alkyl acrylate in which the n-alkyl group contains x carbon atoms, e.g. C16A is hexadecyl acrylate. |
| CxMA | an n-alkyl methacrylate in which the n-alkyl group contains x carbon atoms, e.g. C16MA is hexadecyl methacrylate. |
| (meth)acrylate | an acrylate or methacrylate |
| (meth)acrylic acid | acrylic acid or methacrylic acid |
| AA | acrylic acid |
| MA | methacrylic acid |
| GA | glycolic acid |
| HEA | 2-hydroxyethyl acrylate |
| LA | lactic acid solution (85+ %) |
| PLGA | Copolymer of GA and LA |
| PTSA | para toluene sulfonic acid |
| SUCAN | succinic anhydride |
| SORB | sorbitol |
| AIBN | azo bisisobutyronitrile |
| DCM | dichloromethane |
| TCM | trichloromethane (chloroform) |
| PEG₆ A | CH₂=CH-CO-(OCH₂CH₂)₆-OCH₃ |
| PEG₆ MA | CH₂=C(CH₃)-CO-(OCH₂CH₂)₆-OCH₃ |
| 2-HEMA | CH₂=C(CH₃)-CO-OCH₂CH₂-OH |
| IPA | Isopropyl alcohol |
| MCR | monomethacryloxypropyl alkyl polydimethylsiloxane wherein the alkyl group contains 1-8 carbon atoms. sold by Gelest as MCR-M17 |
| BMP | *butyl-3-mercapto propionate* |
| Trigonox | *Trigonox 42S - t-butyl peroxy-3,5,5-trimethyl hexanoate* |
| Dexa | *Dexamethasone, which has a solubility parameter of 20.85, and which is (9-fluoro-11β,17,21-trihydroxy-16a-methylpregna-1,4 -diene-3,20-dione)* |
| Prav | *Pravachol, which has a solubility parameter of 21.07, and which is (3,5-dihydroxy-7-[6-hydroxy-2-methyl-8- (2-methylbutanoyloxy)- 1,2,6,7,8,8a-hexahydronaphthalen-1-yl]- heptanoic acid)* |
| Risp | *Risperidone, which has a solubility parameter of 17.48 and which is (4-[2-[4-(6-fluorobenzo[d]isoxazol-3-yl)-1-piperidyl]ethyl]-3-methyl-2,6-diazabicyclo[4.4.0]deca-1,3-dien-5-one)* |
| Dcl | *Diclofenac sodium, which has a solubility parameter of 23.4 and which is (2-[2-(2,6-dichlorophenyl)aminophenyl]ethanoic acid)* |
| FTIR | *Fourier transform infrared spectroscopy* |
| *Boc-protected* | *an amine group which has been reacted with di-tert--butyl dicarbonate so that it is no longer reactive.* |

### ECC Polymers

### Crystallinity and Heat of Fusion of ECC Polymers

The crystallinity which is an essential part of the ECC polymers is provided by association of the Cy moieties with each other and/or with moieties in other materials (which moieties may also be Cy moieties). The Cy moieties comprise moieties which can overlap and interact with each other and/or other moieties to form crystalline aggregates or domains. Many other examples of such moieties are disclosed below. The extent of the crystallinity depends upon the ability of such moieties to overlap and interact. Crystallinity is, therefore, increased by increasing the proportion of Cy moieties, by increasing the proximity of the Cy moieties to each other (for example by placing all or a large proportion of the Cy moieties in a block (e.g. a grafted block) of a copolymer, or in a terminal unit of a polymer or compound), and by minimizing other moieties which are bulky enough to interfere with the ability of the Cy moieties to overlap and interact. The value of the heat of fusion, ΔH, of an ECC polymer reflects the extent of its crystallinity, and is at least 5, for example at least 10, at least 20, at least 25, at least 30, at least 35, at least 40, or at least 45, J/g., and may be for example with 10-50, 10-40, 15-35, 3-30, 3-22, or 3-10, J/g.

### Tp, Melting Range (Tp-To) and Mn of ECC polymers

The melting point (Tp) of the ECC polymers is primarily dependent on the nature of the Cy moieties, and (unlike main chain crystalline polymers) is not highly dependent on the molecular weight of the carrier. The ECC polymer has a Tp of at least 40°C, e.g. 40-47°C. ECC polymers with a Tp higher than 70°C, may be useful in certain embodiments. The fact that the molecular weight can be controlled with relatively little change in Tp and Tp-To is important. It means that it is possible, by selection of the Cy moieties (and other moieties) in the ECC polymer, and the method used to prepare the ECC polymer (e.g. making use of chain transfer agents when preparing a polymer), to make a polymer which has a desired Tp, Tp-To and molecular weight. These measures can also be used to control the melting range of the ECC polymers. The melting range can conveniently be quantified by the value of Tp-To (To being the onset of melting temperature, as defined above). The ECC polymer has a Tp-To < Tp^{0.7}, e.g. < Tp^{0.6}; for example, if Tp is 40°C, Tp-To is less 13.2°C, e.g. less than 9.1°C. In other embodiments, Tp-To is less than 15°C, less than 10°C or less than 5°C. The ability to control these variables is valuable for various purposes. For example, in the case of a pharmaceutical formulation, Tp can be selected with reference to in vivo temperatures. In addition, Tp and Tp-To can be selected to facilitate the preparation and processing of formulations at relatively low temperatures, particularly when the drug would be degraded by higher temperatures.

Generally, the Tp of an ECC polymer increases as the number of linear carbon atoms in the Cy moieties increases.

The Mn of the ECC polymer can influence the incorporation and/or retention and/or delivery of a drug. Mn is less than 10,000, and can be less than 8000, or less than 7000, or less than 5000, or less than 2500, or less than 1000, e.g. 1,000-10,000

### b Moieties

b may for example be a covalent bond, or a divalent organic moiety (e.g. an aliphatic, aromatic or mixed aliphatic/aromatic moiety) or inorganic moiety. Examples of b moieties include ester (i.e. -COO -), carbonyl, amide, amine oxide, hydrocarbon (for example phenylene), amino, ether, polyoxyalkylene, and ionic salt linkages (for example a carboxyalkyl ammonium, sulfonium or phosphonium ion pair).

### Cy Moieties

The Cy moieties in a particular ECC polymer may be the same or different. The Cy moieties must be such that they are capable of interacting with other Cy moieties, for example other Cy moieties elsewhere on the same polymer and/or on a different compound, which may be a polymer (which may or may not be an ECC polymer) and/or on a non-polymeric compound, to provide crystallinity. The interaction between the Cy moieties is generally through hydrogen bonds or Van der Waals forces, rather than via covalent or ionic bonding.

Specific examples of suitable Cy moieties include n-alkyl moieties containing 18, 20 and 22 carbon atoms.

### Cy moieties containing polyoxyalkylene moieties

Some useful Cy moieties include polyoxyalkylene, e.g. polyoxyethylene, units. Such a Cy moiety can for example be derived from alkoxy polyoxyalkylene(meth)acrylates, where the alkyl portion of the alkoxy group is an n-alkyl, group containing 18-24 carbons, and the polyoxyalkylene unit is a homopolymer, random copolymer, or block copolymer containing 2 to 100, e.g. 5 to 100, preferably 5 to 60, oxyalkylene units, preferably 2-20, e.g. 2-4, oxyalkylene units. Specific examples of such monomers include stearyl polyethoxylated (meth)acrylate, behenyl polyethoxylated (meth)acrylate, and lauryl polyethoxylated (meth)acrylate. The polyoxyalkylene unit can be attached to the alkyl side chain portion, as for example in hydroxypolyalkyleneoxyalkyl (meth)acrylates with similar alkyl and polyalkyleneoxy groups as above, e.g. hydroxypolyethleneoxystearyl acrylate, hydroxypolyethyleneoxycetyl methacrylate and the like.

### ECC polymers in the Form of Emulsions

In some embodiments, the ECC polymer is in the form of an emulsion. The average size of the particles in the emulsion (and preferably the maximum size of substantially all particles) is preferably less than 1200 nm, e.g. less than 800 nm or less than 500 nm, for example less than 200 nm or less than 100 nm (0.1 µ). In many embodiments, the size of emulsion particles is 50-200 nm, or 50-500 nm, or 100-1000 nm. Some such emulsions can be prepared using the techniques described in US Patent Nos. 6,199,318 and 6,540,984. Emulsions are for example useful for formulations to be injected, generally intravenously, but in some embodiments, intramuscularly or into other tissues. In general, injectable emulsion particles have a diameter of less than 800 nm, or they can be less than 20 p, or less than 10 p, or less than 1 µ in size.

### Mixtures of ECC polymers

A single ECC polymer or a mixture of ECC polymers can be used. The ECC polymer or polymers can also be mixed with an additional material, for example a polymer which is not an ECC polymer. Examples of such additional materials include main chain crystalline polymers, and bioerodable polyesters. Specific examples of such other polymers include poly(epsilon-caprolactone (PCL), poly(dioxanone), polyhydroxybutyrate (PHB), polyhydroxyvalerate (PHV), polyhydroxyalkanoates (PHAs), polyesters from 3-hydroxypropionic acid, polymers derived from methylene carbonate, polyanhydrides, polyorthoesters, naturally occurring polymers or their hydrolysis or degraded products such as sugars, hydrolyzed starches, hyaluronan (also called hyaluronic acid or hyaluronate), chitan, chitosan, and alkyl polylactides, including those disclosed in WO 2007/0142461 (Baker et al.), US 6,469,133, US published application Nos. 20070142461 and 20010044514.

The criteria for the selection of a particular ECC polymer or mixture of ECC polymers, and optionally one or more additional polymers, depend upon the release material and its desired loading and/or release, as further discussed below. Some embodiments of the invention make use of a formulation containing a mixture of two or more ECC polymers having substantially different Tps, for example Tps which differ from each other by at least 2°C, or at least 4°C or at least 6°C or at least 8°C or at least 10°C. Other embodiments make use of mixtures of ECC polymers having a drug bound through a range of ionic strengths as defined by the pKa or pKb of the drug-polymer pair.

In the ECC polymers containing more than one terminal unit of formula (2), and/or a terminal unit containing two or more Cy moieties, for example a total of 2, 3, 4 or 5 Cy moieties in one or more terminal units of formula (2), one or both of b and Cy can be the same or different in the different terminal units, and in a terminal unit containing more than one Cy moiety, the Cy moieties can be the same or different. The ECC polymer can optionally contain, in addition to the units of formulas (1) and (2), repeating units and/or terminal units having a different formula. For example, the repeating units can be derived from a mixture of lactic acid and glycolic acid, and the polymer can contain two terminal units of formula (2), each containing at least one Cy moiety, e.g. an n-alkyl moiety containing 18 carbon atoms.

These preferred ECC polymers can for example be prepared by endcapping a preformed PLGA by (i) reaction of the carboxyl end group of the PLGA with an alcohol containing a Cy moiety, e.g. stearyl alcohol or behenyl alcohol, or (ii) reaction of the carboxyl end group of the PLGA with a polyhydroxy compound, e.g. a sugar such as sorbitol, followed by esterification of one or more of the remaining hydroxy groups with a carboxylic acid (or the like) containing a Cy moiety, and/or (iii) by reaction of the hydroxyl end group of the PLGA with an n-alkyl carboxylic acid (or the like) in which the n-alkyl moiety contains 18-22 carbon atoms.

These preferred ECC polymers can consist essentially of the moieties of the formulas (1) and (2), i.e. the polymer is derived from a PGA (in which each of F¹ and F² is hydrogen in each of the repeating units), a PLGA (in which each of F¹ and F² is hydrogen in some of the repeating units, and one of F¹ and F² is hydrogen and the other of F¹ and F² is methyl in the other repeating units) or a PLA (in which one of F¹ and F² is methyl and the other of F¹ and F² is hydrogen in each of the repeating units). Alternatively, the polymer can also contain repeating units of a different type, optionally providing further carboxy linkages in the polymer backbone, for example units derived from caprolactone.

The following statements disclose optional characteristics of these preferred ECC polymers. Two or more of the stated characteristics can be present at the same time, except where the context makes this impossible.

### (A) the polymer

(A1) has a molecular weight less than 8,000, or less than 7,000, or less than 5,000;
(A2) has a value of (Tp - To) which is less than Tp^{0.6}, or less than 10°C;
(A3) contains less than 30%, e.g. less than 20% or less than 10%, and/or more than 1 %, e.g. more than 2% or more than 4%, molar percent of units comprising Cy moieties;
(A4) has a heat of fusion of at least 10, or at least 20 J/g;
(A5) at least 50 mol percent, e.g. at least 70 mol% or at least 80 mol%, of the repeating units forming the backbone of the polymer are free of Cy moieties;
(A6) the terminal units have one or more of the formulas (2A)-(2C) below

   (2A) -Cy

   (2B) -CO-O-Cy

   (2C) -R_{pbalc}-CO-CY

   where R_{pbalc} is the residue of a polyol, for example a polyethylene glycol, 1, 3-propanediol, glycerin or sorbitol, and optionally contains one or more Cy moieties, the terminal and other Cy moieties, if any, being introduced for example by esterification, transesterification or alkylation of one or more of the hydroxyl groups, e.g. by reaction with an acid or acid chloride containing a Cy moiety; and has for example the formula

   -CH₂-CH(OH) -CH₂-O-CO- Cy

   or

   -CH₂-CH(O-CO- Cy) -CH₂- O-CO- Cy;
(A7) contains less than 170 repeating units of formula
   -CF¹F²-CO-O-;
(A8) is prepared by a process which comprises copolymerizing one or more monomers, i.e. one or more of lactic acid and glycolic acid, and their cyclic dimers, lactide and glycolide, which will result in the repeating units of formula (1) and one or more monomers or components which will result in the terminal units which have formula (2) or which can be converted into terminal units of formula (2). For example, (i) an alcohol containing a Cy moiety can be reacted with lactic acid and glycolic acid, or (ii) a polyol, for example glycerine, 1,3-propanediol or 1,6-hexanediol, can be reacted with glycolic acid and lactic acid to form a PLGA glyceride ester which is end capped with an excess of a carboxylic acid containing a Cy moiety, for example stearic acid or behenic acid.;
(A9) is prepared by endcapping a preformed polymer having the formula HO-(-CF¹F²-CO-O-)ₙ-H where n is an integer less than 170; the preformed polymer, if prepared by the polymerization of lactide and glycolide, will consist predominantly of pairs of identical repeating units, and if prepared by the polymerization of the monomers (lactic and glycolic acids) will have randomly distributed repeating units; for example
   (A9a) is prepared by a process which includes the step of reacting the terminal hydroxyl group of a preformed PLA, PLGA or PGA polymer with a monomer or component, e.g. a carboxylic acid or acid chloride which contains a Cy moiety, or with a monomer or component which contains a moiety which can be further reacted so that it comprises a Cy moiety; or
   (A9b) the polymer is prepared by a process which includes the step of reacting the terminal carboxyl group of a preformed PLA, PLGA or PGA polymer with a component, e.g. an alcohol, which contains a Cy moiety, or with a component which contains a moiety which can be further reacted, e.g. by transesterification, so that it comprises a Cy moiety. For example, the preformed polymer can be reacted with (i) an alcohol containing a Cy moiety, or (ii) a polyol, for example glycerine, 1,3-propanediol or hexanediol, followed by reaction of one or more of the remaining hydroxyl groups of the polyol with an excess of a carboxylic acid containing a Cy moiety, for example stearic acid or behenic acid; or
   (A9c) is prepared by a process which includes the step of reacting the terminal hydroxyl group of a preformed PLA, PLGA or PGA polymer with a dicarboxylic acid or anhydride, e.g. succinic anhydride or succinic acid (succinylation); the resulting product, which may have a relatively high molecular weight in which case it may be in the form of a gel, facilitates formulation mixing in-situ, and/or may enable easy mixing of a protein or peptide drug at one pH and release at another pH; or
   (A9d) is prepared by reacting a preformed PLA, PLGA or PGA polymer with methacrylic anhydride and this reaction mixture is copolymerized with an ethylenically unsaturated monomer containing a Cy moiety; or
(A10) contains at least 10%, e.g. 10-40%, by weight of the Cy units.

### Preparation of ECC polymers

The ECC polymers can be prepared in any way, for example using techniques which are well-known to those skilled in the art. For example, the ECC polymers can be prepared by emulsion, solution, bulk and suspension polymerization techniques using conventional catalysts. Conventional additives and catalysts can be employed to achieve desired molecular weights. The type of polymerisation can often be selected according to the form of pharmaceutical formulation to be administered.

### Drugs

A wide variety of drugs can be delivered through the use of this invention. The ECC polymer can be associated with one or more hydrophilic or hydrophobic drugs, for example, a small molecule, polypeptide, protein, carbohydrate, polynucleotide, nucleoside, siRNA, immunoglobulin or Fc or Fab fraction thereof, a cyclic compound, alkaloid, beta-lactam, or other antibiotic. The drug may also be an agonist or antagonist of neurotransmitters or hormones, an antipsychotic (for example fluphenazine maleate, chlorpromazine, chlorpromazine hibenzoate, sulpiride, carpipramine hydrochloride, carpipramine maleate, clocapramine hydrochloride, mosapramine hydrochloride, Risperidone (or any compound containing functional groups of benzisoxazole and/or piperidine), clozapine, olanzapine and sertindole and flupenthixole and perphenazine); or an SSRI; or it may comprise cell signalling molecules such as cytokines such as lymphokines, monokines, chemokines, interleukins, prostoglandins etc a statin, a Cox-2 inhibitor, an SSRI, a calcium channel blocker, psychotropic drug, bisphosphonate, anti-proliferative, mitotic inhibitor, angiogenics , antiangiogenics, small molecule such as rapamycin or derivatives, antipain medications, anti-inflammatories, hormones, anti-osteoporosis drugs, cytotoxic agents, chemotherapeutics and contraceptives. Specific drugs and drug classes include the following, and precursors and pharmacologically active derivatives, congeners, and metabolites thereof:risperidone, olanzapine, atorvastatin, celecoxib, omeprazole, esomeprazole, quetiapine, metoprolol, budesonide, ibuprofen, uracils (eg., 5-fluorouracil), steroids and esters of steroids or other hormones (e.g., estrogen, progesterone, testosterone, androsterone, cholesterol, norethinidrone, digoxigenin, cholic acid, deoxycholic acid, and chenodeoxycholic acid), boron containing compounds (e.g., carborane), chemotherapeutic nucleotides, drugs (e.g., antibiotics, antivirals, antifungals), enediynes (e.g., calicheamicins, esperamicins, dynemicin, neocarzinostatin chromophore, kedarcidin chromophore), heavy metal complexes (e.g., cisplatin, carboplatin), hormone antagonists (e.g., tamoxifen), non-specific (non-antibody) proteins (e.g., sugar oligomers, insulin), polynucleotodes and oligonucleotides (e.g., mRNA sequence), radio-nuclides, toxins (e.g., ricin, and transcription based pharmaceuticals. In certain embodiments the drug may be or may contain bacterial toxins such as botulinum toxin ("botox").

The table below gives a selection of some drugs that can be incorporated into and delivered by the pharmaceutical formulations of the invention.

| Name | Trade Name | Drug Class | Mᵣ/Da | Hydrophobic |
|---|---|---|---|---|
| Atorvastatin | Lipitor | Statin | 1,209 | YES |
| Symvastin | Zocor | Statin | 418 | YES |
| Celecoxib | Celebrex | Cox-2 inhibitor | 381 | YES |
| Sertradine | Zoloft | SSRI | 343 | YES |
| Omeprazole | Losec/Prilosec | PPI | 345 | YES |
| Esomeprazole | Nexium (isomer of Prilosec) | PPI | 767 | YES |

| risperidone | olanzapine atorvastatin | celecoxib | omeprazole | esomeprazole |
|---|---|---|---|---|
| Azithromycin | Zithromax | Antibiotic | 749 | NO |
| Quetiapine | Seroquel | SSRI | 883 | YES |
| Metoprolol | Seloken/Toprol | Beta-blocker | 653 | NO |
| Budesonide | Pulmicort/Rhinocort | Anti-inflammatory-steroid | 431 | YES |
| Clarithromycin | Biaxin | Antibiotic | 748 | YES |
| Paroxetine | Paxil | SSRI | 375 | NO |
| Oxycodone | Oxycontin | Opioid agonist | 351 | NO |
| Risperidone | Risperdal | Psychotrope | 410 | YES |
| Alendronate | Fosamax | bisphosphonate | 325 | NO |
| Venlafaxine | Effexor | SSRI | 314 | NO |
| Amlodipine | Norvasc | Ca-channel-blocker | 567 | YES |
| Olanzapine | Zyprexa | | 312 | YES |
| Lansoprazole | Prevacid | PPI | 369 | YES |
| Fluoxetine | Prozac | SSRI | 345 | NO |
| Finasteride | Proscar | steroid-antagonist | 372 | YES |
| Sildenafil | Viagra | PDE5-antagonist | 667 | NO |
| Rosuvastatin | Crestor | Statin | 1001 | YES |

The drugs that can be used include chemotherapeutic agents used to kill neoplastic cells, for example (1) Alkylating agents, which destroy the cancer cell's DNA e.g., Busulfan, Cyclophoshamide and Melphalan, (2) Nitrosoureas, that inhibit a cancer cell's enzymes needed for DNA repair, e.g., Carmustine and Lomustine, (3) Anti-metabolites, that interfere with both a cancer cell's DNA and RNA, e.g., 5-Fluorouracil, Methotrexate and Fludarabine, (4) Anti-tumor antibiotics that interfere with a cancer cell's DNA in addition to changing its cellular membrane, e.g., Bleomycin, Doxorubicin and Idarubicin, (5) Mitotic Inhibitors, that inhibit enzymes needed for protein synthesis in the cancer cell, e.g. Docetaxel, Etoposide, Vincristine and Vinblastine and Vinorelbine, and (6) Radioactive isotopes, which destroy cancer cells by the production of radioactive emissions, e.g., indium-111 (¹¹¹In), yttrium-90 (⁹⁰Y).

The drugs that can be used for sustained release applications include, for example, anti-pain medications (e.g. morphine analogues), anti-psychotics (e.g. risperidone), anti-inflammatories (e.g. steroids or NSAIDs such as Budesonide), hormones (e.g. testosterone or progesterone) cholesterol lowering drugs (e.g., statins), osteoporosis drugs (biphosphonates), anti-angeogenics (e.g., anti-VEGF) and contraceptives.

The drugs they can be used include the derivatives (defined herein as including, but not limited to any pharmacologically active metabolite, or analogue, agonist, derivative, variant, congener, or isomeric form) of any drug described herein.

### Ligand-Targeted Polymeric Drug Delivery

In certain embodiments, the ECC polymer incorporates ligands that bind specifically to target receptor molecules. Target receptor molecules are present on the surface of a specific type of target cell, such as a cancer cell. The ligands may be incorporated into the polymer formulation by copolymerization of a comonomer which carries a receptor covalently attached to the monomer. Alternatively a ligand may be incorporated into the pharmaceutical formulations of the invention by any other means which does not involve covalent bonds and which allows the ligand to associate with the polymer, either as a simple physical mixture, or by ionic bonds, hydrogen bonds, Van der Waal's forces or hydrophobic/hydtrophilic interactions. The ligands bind specifically to molecules expressed on the cell surface of a target e.g., receptor proteins that are differentially over-expressed on target cells, for example cancerous cells.

For example, many types of cells express a large number of folate receptors on their cell surface, more so than for non-cancerous cells. Folic acid or derivatives or portions of folate can be physically mixed into the pharmaceutical formulation during its preparation, or added to the outside of particles formed after crystallization or precipitation of the formulation. The folate molecules bind specifically with the folate receptors on a target cancer cell. A chemotherapeutic agent, for example carboplatin or cis-platin or taxol, can be mixed with or bound by hydrogen bonding or otherwise to the ECC polymer so that it can thereafter be delivered to a specific cell target.

This ligand-targeted polymeric drug delivery system can be used to deliver known targeted cancer therapy agents. For example, small-molecule drugs can be used to block specific enzymes and growth factor receptors (GFRs) involved in cancer cell growth. These drugs act as signal-transduction inhibitors. One such drug that may be used with the invention is Gleevec ® (STI-571 or imatinib mesylate) to treat gastrointestinal stromal tumours and certain kinds of chronic myeloid leukemia. Another drug that may be used is Iressa® (ZD1839) that is used to treat advanced non-small cell lung cancer. This drug targets the epidermal growth factor receptor (EGFR), which is overproduced by many types of cancer cells.

Apoptosis-inducing drugs may also be incorporated . One such drug is Velcade® used to treat multiple myeloma. Velcade blocks proteasomes, which help to regulate cell function and growth. Other apoptosis-inducing drugs include Genasense™ to treat leukemia, non-Hodgkin's lymphoma, and solid tumours. Genasense blocks the production of BCL-2, which promotes the survival of tumor cells.

Other possible drugs include monoclonal antibodies that bind to growth factor receptors, such as Herceptin® (Trastuzumab).

A number of other tumor-specific targets are discussed below. Many of the ligands describe below are either small proteins (about 30-50 amino acid residues) or have a small active site of only a few residues (often 3-7 amino acids) and can be attached to the ECC polymers used in this invention, for example covalently.

The epidermal growth factor receptor (EGFR) is over-expressed in the majority of colorectal cancers. Also expressed in the gut are gastrointestinal (GI) peptide hormones, such as gastrin, CCK, secretin, glucagon, somatostatin, and their cognate G protein-coupled receptors (GPCRs). EGFR, Gl hormones or any related proteins may be used as specific targets by providing a formulation with a ligand, such as a specific antibody, that binds specifically to EGRF or Gl hormones or homologous proteins or portions thereof.

A number of leukemias and lymphomas, including cutaneous T-cell lymphoma, chronic lymphocytic leukemia, and B-cell non-Hodgkin's lymphoma, express the interleukin-2 receptor (IL-2R) receptor. Antibodies or other ligands (e.g., DAB389IL-2, ONTAK™; Seragen Inc, San Diego, CA) targeting the interleukin-2 receptor may be used to target a polymer-drug formulation of the invention. Monoclonal antibodies directed against CD33, a cell surface antigen highly expressed in acute promyelocytic leukemia (APL) may be used as a targeting ligand in the present invention against APL tumor cells.

Rituximab is an anti-cancer monoclonal antibody that binds to CD20 which is expressed on the surface of malignant B cells. Rituximab may be used as a targeting ligand in the present invention.

Antibodies and other ligands that bind OPG or RANKL can be used as targeting ligands in the present invention against multiple myeloma tumor cells.

The expression patterns of folate receptor (FR) isoforms, alpha and beta, in normal and malignant male and female reproductive tissues are well studied. Folate, analogues of folate and other ligands that bind to folate receptors can be incorporated into the pharmaceutical formulations of the invention to provide specific and/or preferential targeting of various tumor tissues.

Prostate-specific membrane antigen (PSMA) is a metallopeptidase expressed predominantly in prostate cancer (PCa) cells. Ligands that bind to PSMA can be incorporated into the pharmaceutical formulations of the invention to provide specific and/or preferential targeting of prostate tumor tissue.

Two protein hormones having activities that are similar to insulin are termed insulin-like growth factor I (IGF-I) and IGF-II. In parallel, there are two receptors, the insulin-like growth factor 1 receptor (IGF1 R) and IGF2R that bind with differing affinities to insulin, IGF-I, and IGF-II. IGF2R acts as a cell surface receptor for many proteins relevant to breast cancer biology, including IGF-II. IGF2R could be targeted using sugar moieties and/or polysaccharides attached to the ECC polymers used in this invention.

Ovarian cancer G protein coupled receptor 1 (OGR1) and GPR4, two structurally related receptors are high affinity molecular targets for SPC. Such receptors for phospholipids on tumors would be ideal targets for polymers as used by the invention coupled to lysophosphatidic acid (LPA), sphingosylphosphorylcholine (SPC), and/or phosphatidic acid (PA). Ligands such as phospholipids that bind to these targets may be incorporated into the pharmaceutical formulations of the invention to provide specific and/or preferential targeting of ovarian and other tumor tissue.

Another specific target molecule is fibroblast growth factor (FGF). Activated FGF initiates a kinase cascade resulting in phosphorylation of docking protein fibroblast growth factor receptor substrate 2 (FRS2α). Anti-FGF antibodies of portions of FRS2 domains may serve as ligands for targeting tumors. Programmed death receptor ligand 1 (PD-L1) is abundant on many cancers. Ligands that bind to PD-L1, such as antibodies or IFN-gamma or related molecules could provide specific and/or preferential targeting of cancer cells and tissues.

The glycolipid-anchored receptor for urokinase-type plasminogen activator (uPA) is overexpressed at the invasive tumor-stromal microenvironment in many human cancers. Ligands that bind to uPA, such as antibodies could be incorporated into the formulations of the invention to provide specific and/or preferential targeting of cancer cells.

CXCR4 is a seven-span transmembrane protein that is a native receptor for the alpha-chemokine stromal-derived factor-1 (SDF-1) that is expressed on the surface of cancer stem cells. The SDF-1-CXCR4 axis is also involved in directing their trafficking/metastasis to organs that highly express SDF-1 (e.g., lymph nodes, lungs, liver, and bones). A region of SDF-1 homologous to the receptor-interacting region could be incorporated into the pharmaceutical formulations of the invention to provide specific targeting to cancer cells, particularly to treat or prevent metastasis of tumor cells.

Notch signalling contributes to pre-malignant metaplastic changes that precede pancreatic carcinoma, and it is also likely to be involved in other forms of metaplasia. The Notch receptor ligand can be incorporated into the pharmaceutical formulations of the invention to provide specific targeting to pancreatic carcinoma and other cancer cells.

Additionally, other types of ligands may be incorporated into the formulation of the invention including ligands that recognize Receptor Protein Kinases, G-Protein Coupled Receptors, Nuclear Receptors, Ligand-Gated Receptor Ion Channels, Macrophage Scavenger Receptors, T-Cell Receptors, Netrin Receptors, VPS10 Domain Containing Receptors, Tetraspan Family Proteins, Ion Channels, ABC Transporters, Semaphorins and Neuropilins, Membrane Proteins Associated with Intercellular Communication and Peripheral and Anchored Membrane Proteins.

In many of the above cases, the ligand may be an antibody or portion of an antibody (such as the Fab portion or fragment thereof) that binds specifically to the cancer-associated target molecule. Antibodies may be polyclonal or monoclonal and often may be humanized. Such antibodies can be produced by standard well-known methods. Monoclonal antibodies (MAb's) that bind specifically to tumor-associated antigens have been employed in an attempt to target toxin, radionucleotide, and chemotherapeutic conjugates to tumors. To date, a variety of monoclonal antibodies have been developed that induce cytolytic activity against tumor cells. For example, a humanized version of the monoclonal antibody MuMAb4D5, directed to the extracellular domain of P185, growth factor receptor (HER2), is used to treat human breast cancer. Also, a chimeric antibody against CD20, which causes rapid depletion of peripheral B cells, including those associated with lymphoma, is used to treat low-grade B-cell non-Hodgkin's lymphoma. Other humanized and chimeric antibodies under development or in clinical trials include an anti-CD33 antibody that binds to CD-33, which is expressed on most types of myeloid leukemic cells. Another is a chimeric antibody against the leukocyte antigen CD-45 (cHuLym3). The formulations of the invention may incorporate any target-specific ligands of any kind, including the antibodies and ligands mentioned herein, and any derivatives and homologues thereof.

The pharmaceutical formulations of the present invention optionally have one or more of the following characteristics: --
(1) The formulation, in vivo, when implanted within a human subject, continuously releases a therapeutically effective dose of drug to the subject over a period of at least 30 days.
(2) The formulation, in vivo, when implanted within a human subject, continuously releases between 0.1 milligram and 100 milligrams of drug per day over a period between 12 hours and 30 days following implantation.
(3) The formulation, in vitro (elution into 1 L phosphate buffered saline (PBS) the PBS being at a pH of about 7.4, at 37°C, stirred at 100 rpm, or in one of the test procedures described in the Examples below, (i) has an average rate of release of drug from the formulation which is no greater than 20 milligrams per day averaged over any 24 hour period during the first 168 hours of elution, and/or (ii) continuously releases between 0.1 milligram and 20 milligrams per day, averaged over any 24 hour period, over a period of at least 30 days, and/or (iii) releases no more than 20% by weight of drug over a period of 24 hours, and/or (iv) releases the drug at an average rate no greater than 25 milligrams per day averaged over any 24 hour period during a period from 12 hours to 168 hours following inception of elution.
(4) The formulation is adapted for oral delivery and the drug has not more than 5, preferably not more than 10, hydrogen bond donors.
(5) The formulation is adapted for oral delivery and the drug has a molecular weight under 500 g/mol.
(6) The formulation is adapted for oral delivery and the drug has a LogP (partition coefficient) under 5.
(7) The formulation, in vivo, when implanted subcutaneously or intramuscularly within a human subject, continuously releases a therapeutically effective dose of the drug to the subject over a period of at least 30 days.

### Specific Embodiments of Pharmaceutical Formulations

One specific example of a formulation containing the psychoactive (antipsychotic) agent Risperidone, (4-[2-[4-(6-fluorobenzo[*d*]isoxazol-3-yl)-1-piperidyl]ethyl]-3-methyl-2, 6-diazabicyclo[4.4.0]deca-1,3-dien-5-one) is the following. Risperidone may be physically mixed with an ECC polymer, either directly, or after it has been pre-associated, for example with a surfactant, PEG, human (or bovine etc) serum albumin, or with proteins etc. to provide immunological shielding, increased half-life, and improved bioavailability. The formulation may be formed into a shaped solid implant suitable for introduction, e.g. by trocar, under the skin or intramuscularly or by injection above the formulation's melting temperature. It is believed that a dose of Risperidone of about 4 mg/day is sufficient to prevent psychotic episodes in many patients. Individual variations in tolerance and effectiveness, however, can be wide. Therefore the current invention may be formulated to supply from 1 to 60 mg/day over a period of 1 to 200 days. The advantage of an implanted dosage form is increased compliance, which with psychosis, is a major issue. In one embodiment, at least 50 mg of Risperidone is formulated into a single implantable dosage form by mixing the drug in powdered form with an ECC polymer at a temperature above the Tp of the polymer, for example at between 42ºC and 60ºC. In some cases, no solvent is required. In other cases, a solvent is used to improve compatibility and is subsequently removed. The mixture is cooled and shaped into, for example, solid elongated or approximately spherical implants. In other embodiments, the amount of Risperidone formulated into a single implantable dosage form may be at least 100 mg, or at least 200 mg, or at least 500 mg, or at least 1000 mg, or at least 1500 mg, or at least 2000 mg. In some dosage forms, the total amount of drug may be up to 3, 4 or 5 grams. The Risperidone implant is introduced subcutaneously into a subject using a trocar or minor incision. The implant releases Risperidone at an average rate of between 1 and 60 mg (for example no more than 1, 2, 3, 4, 5, 7, 10, 20, 40, 80, 120, 200, or 300 mg) per day over a period of at least 5, 10, 15, 20, 25, 30, 40, 60 or 90 days. In one exemplary embodiment the implant releases Risperidone at an average rate of between 4 and 12 mg per day over a period of at least 7 days. During this period, a desired therapeutic effect is provided for at least 75% of the time. The implant is then removed, or may be left in to erode over time. If a larger bolus of drug is desired, the local area of skin above the implant may be heated by any convenient means.

In other embodiments the implant releases Risperidone at an average rate of between 1 and 20 mg per day, or 3 and 100 mg per day or 4 and 30 mg per day over a period of at least 7 days or at least 14, 21, 30, 45, 60 or 90 days. The Risperidone is delivered in such a manner and rate that the blood plasma level of risperidone is within the human therapeutic window, 1 to 125 ng/ml over the period of 30 or 60 or 90 days.

In certain related embodiments, Risperidone may be pre-associated with a surfactant, PEG, human serum albumin or with proteins. In another embodiment, the CYSC polymer comprises side chains having an average length of, for example, between 6 and 50 monomer units. In another embodiment, the CYSC polymer has side chains with an average length of between about 12 and 50 alkyl ester acrylate or methacrylate monomer units.

In certain embodiments, Risperidone is physically mixed with the ECC polymer and no covalent or ionic bonds are formed between the drug and the polymer. In some embodiments, the drug associates with the polymer via hydrogen bonds and/or van der Waals forces.

In another embodiment designed to release a drug to control pain, the drug of the formulation may be an opioid, opioid antagonist, synthetic opioid, morphine, fentanyl or sufentanyl, or any type of local or systemic analgesic acting on peripheral or central nervous pair receptors. In such embodiments, the polymer may be implanted under the skin such that the drug is released over a period of time to treat pain for a number of hours such as at least 3, at least 6 or at least 12 hours. The formulation may also be an oral formulation, transdermal formulation or depot.

### Formulations, Methods of Making Formulations and Methods of Delivering Drugs

The formulations of the invention comprise an ECC polymer and a drug associated therewith. In some embodiments, the drug is dissolved in the ECC polymer, thus forming a single phase. In other embodiments, the drug is uniformly or non-uniformly distributed as a separate phase in the ECC polymer, for example as a dispersion or emulsion.

In some embodiments, the formulation is a single phase formulation. In other embodiments, the formulation comprises at least two phases, for example comprising a matrix in which the ECC polymer, and drug are uniformly or non-uniformly distributed as a separate phase (which, as indicated above, can comprise a solution of the drug in the ECC polymer, or a uniform or non-uniform distribution of the drug as a separate phase in the ECC polymer.

The Tp of the formulation can be the same as the Tp of the ECC polymer, but is more often somewhat lower than the Tp of the ECC polymer. The Tp of the formulation can for example be 40-47°C.

The formulation can contain any appropriate proportion of the drug, depending on the intended use of the formulation. In some embodiments, the weight of the drug, based on weight of the formulation is at least 1%, at least 2%, at least 5%, at least 7%, at least 8%, at least 10%, at least 15%, at least 17%, at least 20%, at least 25%, at least 30%, at least 35%, at least 40%, at least 45%, at least 50%, at least 55%, at least 60%, or at least 70%, e.g. 1-20%, 5-30%, 10-30%, 20-30%, 10-50%, or 20-50%. In other embodiments, the formulation contains 0.1-5%, for example up to 2%, up to 3%, or up to 4%, of the drug. High drug loading is particularly useful for sustained release formulations. In some embodiments, the present invention combines high drug loading with reduced burst effect.

The ECC polymer, should be selected having regard to the nature of the drug, the way in which the formulation is to be applied to the target site, and the way in which the drug is to be released. For example, the drug may be delivered as an oral tablet or capsule, as a parenteral subcutaneous injection or intravenous infusion, as a transdermal patch application, as an implant which may later be explanted, as a rectal administration, by pulmonary techniques or as a drug-eluting coating on a medical device, e.g. a drug-eluting stent. Dependent on the application, the formulation may most appropriately be in the form of a solid, a solution, a coating, a particle (e.g. a microparticle or nanoparticle), an emulsion or a suspension.

A simple form of association is a solution of the drug in the ECC polymer. In that case, for example when the solution is a pharmaceutical formulation which is injected, the presence of the ECC polymer will delay only slightly the absorption of the drug in vivo. More often, a pharmaceutical formulation is a dispersion, an emulsion or a suspension in which particles comprising the drug and the ECC polymer are uniformly distributed in an aqueous medium.

In some embodiments, the formulation comprises a colloidal dispersion of particles having a size of 1 nm to 0.5 mm. Such a dispersion can be produced by mixing or sonication or homogenization of an aqueous mixture of the drug and polymer matrix in the presence of a surfactant. The drug and/or the ECC polymer may be dissolved in a solvent which evaporates during the sonication or homogenization process. This process can lead to microparticles having a size of from 0.1 to 1000 microns. These colloidal and emulsion mixtures may be suitable for a variety of applications. For example, microsphere particles have a diameter of 0.1-150, e.g. about 50, µm, suspended in suitable aqueous vehicles may be injected through a conventional syringe using an 18-20 gauge needle. The particle sizes can be measured using a Horiba particle size analyzer LA 910.

The type of drug may influence the processing conditions. For example, some drugs, e.g. proteins, are likely to be adversely affected by conventional emulsification used for microsphere production. This invention can make use of techniques to disperse proteins gently in a polymer solution and atomizing this mixture into liquid nitrogen with extraction of the frozen solvent for the polymer, thus producing stable protein microspheres which are suitable for suspension and injection. This invention can also make use of nonaqueous formulations which maintain protein activity and conformation.

In one method for making formulations comprising an ECC polymer and a drug, the ECC polymer is melted, and the drug is mixed with the ECC polymer, the mixing being carried out at a temperature which is above the To, usually above the Tp, of the ECC polymer. During such mixing, other desired ingredients, e.g. fillers, excipients, dyes, colorings, flavors, disintegrators, stabilizers, can be added. The mixing can optionally be carried out in the presence of another material which is liquid at the mixing temperature and which is not a ECC polymer. This method can result in a uniform mixture which can, if desired, be suspended above the melting point of the mixture in a non-solvent, thus producing, upon cooling, solidification of the formulation as particles. The particles can be washed and filtered, and, for example, suspended in a liquid carrier, e.g. to produce an injectible solution. In another embodiment, in which no solvent is preferably used, the molten mixture, or the solidified mass obtained by cooling the molten mixture, can be processed into desired shapes, e.g. into rods, ovals, and tablets, using known procedures.

Because the ECC polymer can have a relatively low melting point, the ability to carry out mixing at relatively low temperatures is particularly valuable when the drug, e.g. a peptide or protein drug, is a drug which is damaged (e.g. denatured or partially or completely inactivated) by exposure to a temperature which is more than 15°C, or more than 30°C, above the Tp of the ECC polymer. The ECC polymer used in such methods may for example have a Tp about 40°C, 42°C, 45°C, 50°C, 55°C, 60°C, 65°C, 70°C, 75°C or 80°C, for example 40-65°C, 45-60°C, 40-60°C, or 50-65°C. For drug delivery, it is generally desirable that the pharmaceutical formulation should have a Tp above the body temperature of the subject (e.g. 37°C).

In another embodiment, microparticles are prepared by dissolving a ECC polymer in a solvent, dispersing a solid or liquid drug into the solution, and dispersing the mixture rapidly into cold water or spray in the mixture into a chamber, thus forming microspheres or microcapsules.

Another method of making microparticles is the water/oil/water double emulsion method. An aqueous phase containing the drug, for example a protein, is dispersed into an oil phase comprising the ECC polymer, dissolved in an organic solvent under high speed homogenization conditions. The resulting water-in-oil emulsion is then dispersed in an aqueous solution containing a polymeric surfactant such as a polyvinyl alcohol and further homogenized to produce a water/oil/water emulsion. The emulsion is stirred for several hours to evaporate the solvent, and the resulting nanoparticles or microparticles are collected by filtration.

In another embodiment, an ECC polymer is prepared as an emulsion copolymer using emulsion polymerization techniques, optionally in the presence of small amounts of cosolvent. The release material can then be added in a compatible solvent for the emulsion, thus allowing migration of drug into the emulsion polymer particles.

### Peptide and Protein Drug Delivery

Some of the ECC polymers are particularly well suited for the delivery of protein and peptide drugs, including but not limited to, hormones (such as growth hormones and sex hormones), LHRH, insulin, calcitonin, secreted proteins, alpha-glucosidase, interferons, erythropoietin, antibody drug conjugates, Fc fusion proteins, interferon, anti-CD 20 therapeutics, Factor VII, IX or X, monoclonal antibodies, cytokines, kemokines, cell cycle regulators, BSA protein, and transcriptional modulators.

Protein and peptide drugs are large hydrolytically unstable molecules and are often adversely affected by temperature (typically temperatures above about 60°C), pH, acids, enzymes and ionic groupings of time found in the cationic and anionic surfactants which are often used in drug formulations of small drug molecules). Consequently, they are typically administered by subcutaneous injection or intravenous (IV) infusion. It is, therefore, desirable to be able to deliver protein and peptide drugs by other means, for example oral administration, and/or to prolong their release after delivery, thereby reducing the number of injections required for administration. We have found that the problems associated with the present methods of delivering protein and peptide drugs can be mitigated through the use of selected ECC polymers. Examples of such ECC polymers are: --
(1) ECC polymers comprising block copolymers comprising CYSC acrylate blocks and end-capped crystalline PLGA blocks, obtained for example by grafting an end-capped PLGA (ECC PLGA) to a CYSC acrylate), and
(2) mixtures of CYSC acrylates and/or ECC PLGA's, optionally with another Cy-containing material, for example a crystalline fatty acid, a crystalline fatty alcohol, or a crystalline fatty ethoxylate alcohol.

Use of an ECCpolymer can provide
a) Improved loading capability. The ECC polymers in particular the ECC-PLGAs, can be designed to be compatible on mixing with selected drugs, for example peptides and proteins. As a result, high loading can, if desired, be obtained. The ECC polymers can be prepared so that they contain different proportions of units derived from lactic acid and/or glycolic acid (and/or other monomers which can be polymerized to polyesters) in the polymer backbone of the PLGA, and the amount of crystalline end capping can be varied, in order to achieve desired amphiphilic properties, resulting for example in a change in the compatibility, e.g. solubility, of the drug and the ECC polymer.
b) Improved mixing capability. Unlike the polymers presently used, e.g. polyethylene-co-vinyl acetate (EVA) and PLGA, which must have high molecular weights in order to provide acceptable delivery rates, the ECC polymers can have relatively low molecular weights and relatively low melting points. The ECC polymers also contain hydrophilic moieties which assist mixing with the peptide or protein drug. It is often possible, therefore, to mix an ECC polymer with a drug at relatively low temperatures, for example below 75°C, particularly below 60°C. This is particularly desirable when the release material is adversely affected by higher temperatures. ECC polymers can, therefore, be mixed easily with protein and peptide drugs at relatively low temperatures which do not degrade the drug.
c) Improved stability. The presence of the hydrophobic Cy moieties in an ECC polymer enables protein and peptide drugs to be protected from aqueous body fluids, thus preventing bioerosion of the peptide or protein.
d) Repeatable and controllable delivery of drugs, including protein and peptide drugs, with reduced burst, because a stable and uniform mixture of the ECC polymer and drug can be achieved. In some cases, the formulation may be formulated to minimize or substantially eliminate the burst effect and/or to give a zero order, a 1^{st} order or a mixture of a zero and first order release of a drug, including sustained delivery of therapeutically effective levels of drug. Furthermore, in some cases, external heating of the formulation, after it has been delivered to the target site, can result in delivery of the drug at a desired location and/or time.
e) Bioerodability, as described above.

The ECC polymer can be used in combination with other polymers, for example amorphous polymers. For example, the formulation can also contain simple acrylate copolymers which are good film formers, particularly where film-forming properties in addition to drug delivery properties are important, as for example in a medical stent. In some embodiments, it is useful to employ an ECC polymer which includes acid groups to enable oral drug administration of a peptide or protein drug. The Cy moieties protect the drug formulation in the highly acidic stomach environment, but at the high pH in the alkaline upper intestine, the presence of the acid groups causes swelling and release of drug by diffusion, and also by bioerosion.

The presence of acid groups in the ECC polymer may also make it possible to create a fine suspension of the drug/ ECC polymer in a suitable aqueous medium for use in subcutaneous injection. Once injected, the acid-containing ECC polymer swells and forms an "in-situ" gel platform from which the peptide or protein is released through diffusion by pore or channel formation or bioerosion of the bioerodible gel.

In some embodiments, the ECC polymer has a Tp slightly above the normal body temperature of the organism into which the formulation is to be injected. Such a product can for example be administered intravenously, and thereafter activated by some external heat source so that the peptide or protein drug is released at a desired location, e.g. in a cancerous area, adjacent to a tumor. By adjusting the external heating, the release of the drug may be intermittent or bolus release.

### Administration

In one embodiment of the invention, a pharmaceutical formulation of the invention is released in a controlled manner. The method optionally has one or more of the following characteristics
(a) the drug is released over an extended period of time;
(b) no more than 80%, preferably no more than 50%, for example no more than 30%, of the total drug loaded into the formulation is released from the formulation over a period of time of 6 hours following administration of the formulation to a subject;
(c) less than 50%, or less than 10%, of the total drug loaded into the formulation is released over a period of time of 1 hour, or over a period of time of 2 hours, following administration of the formulation to a subject;
(d) at least 75% of the total drug loaded into the formulation is released within 30 minutes of activation of the formulation;
(e) the rate at which the drug is released from the formulation after administration is influenced by (i.e. commenced, increased, reduced or ended) by a change in one or more of the following conditions
   (i) heating or cooling caused by external action, e.g. heating,
   (ii) hydration of the formulation,
   (iii) exposing the formulation to an enzyme,
   (iv) changing the pH of the environment surrounding the formulation.

For example, in some embodiments the formulation releases a therapeutic dose of the drug only at or above a certain pH. For example, a formulation may release little or no drug in an aqueous environment at an acid pH (such as the stomach), but release a therapeutic dose of the drug at a less acid pH such as at pH6, or an alkaline pH, such as pH7, pH8, pH9, or pH10 or above. This application is particularly useful for the oral delivery of acid labile drugs such as proteins or peptides that are damaged by the acid environment of the stomach.

### Drug loading & release

In preferred embodiments, this invention provides one or more of high drug loading, low burst effect, and sustained release of a drug from an ECC polymer formulation tailored to provide the desired characteristics for the particular drug.

Total drug loading is the amount (usually expressed as the percentage by weight) of the drug present in the formulation. The drug may be chemically and/or physically bound to the ECC polymer, for example physically entrapped in the formulation. Binding of the drug to the polymer may be through covalent and/or ionic and/or hydrogen bonding and/or van der Waal's forces and/or hydrophobic interactions.

Many known drug formulations contain less than 5% by weight of drug. For example, the polymers conventionally used for sustained release formulations, such as PLGA (co-polylactic acid-co-polyglycolic acid) and amorphous acrylic copolymers accept only relatively low drug loadings of between about 1 % and 5%. In contrast, preferred embodiments of the present invention provides formulations with relatively high drug loading.

Tailoring drug loading and release properties for a particular drug may be achieved for example through selecting polymers with a combination of desired features relating to (a) ionic or covalent attachment of drug molecules to the ECC polymer, and/or (b) capturing the drug molecules in the crystalline hydrophobic domain, and/or (c) presenting a tortuous path around crystalline domains that drug molecules in the hydrophilic domains must navigate to reach the exterior and/or (d) ensuring that the crystalline domains are preferentially on the outside of the formulation. Methods of preferentially locating the crystalline domains on the exterior include, for example, forming particles in air using a spinning disk allowing the hydrophobic side chains to come to the low energy interface or in a hydrophobic medium that would again draw the hydrophobic side chain to the interface, or actually coating preformed particles.

Drug loading and release properties of ECC polymer formulations can for example be controlled by altering the solubility of the drug in the ECC polymer and/or providing association sites for the drug to interact with the ECC polymer. For example, if the drug is a protein or polypeptide, the ECC polymer can contain ionic groups which are preferably selected from those cationic or anionic groups which naturally associate ionically or through hydrogen bonding with the carboxyl or amide groups of the protein or polypeptide side chains. Such ionic groups can be derived from suitable cationic or anionic comonomers forming part of the ECC polymer can be subsequently prepared on a preformed polymer, for example by quaternization of a tertiary amine moiety on a comonomer. Quaternization can for example be carried out by treatment with a methyl or other alkyl chloride or a dimethyl or other dialkyl sulfate.

In the case of sustained release formulations, several days, weeks or even months of therapeutically effective dosage may be required. A formulation of the invention (such as an implanted drug formulation or device, such as a coated stent or solid implant or polymeric drug depot) may provide such dosage for at least 5 hours, at least 12 hours, at least 24 hours, at least 36 hours, at least 72 hours or at least 100 hours, or even longer, for example at least one week, or at least two weeks, or at least three weeks,

or at least a month or at least three months, or at least six months, or at least a year. In some embodiments, interactions between the drug and the ECC polymer slow the release of the drug from the polymer into the surrounding physiological environment. The Cy moieties of the ECC polymer influence melting behaviour; and once it has melted, the Cy moieties act somewhat like a plasticizer (although they are of course still attached to the remainder of the ECC polymer). As a general rule, and other things being equal, the rate at which a drug will be released will be decreased by (i) an increase in the crystallinity of the ECC polymer, (ii) an increase in the molecular weight of the ECC polymer, and (iii) an increase in the strength of the bonds between the drug and the ECC polymer.

The drug formulations of the invention can be specifically designed to provide controlled and/or sustained release. For example, a drug formulation of the invention may be designed so as to release no more than a certain amount of drug over a specific period of time under certain defined circumstances. The rate of loading and release will depend on the specific drug-polymer pair. For instance, a single dose of a drug formulation may release no more than 5% of the total drug loaded into the dosage form, or alternatively no more than 1 %, 3%, 10%, 20%, 30%, 40%, 50%, 60% or 70% over a period of 1 hour, or alternatively 3 hours, 6 hours, 12 hours, 18 hours, 24 hours, 36 hours, 48 hours, 72 hours or any combination of the foregoing numbers.

The rate of drug released may be approximately zero order during the main period of therapeutic administration, for example over 99% or 95% or 90% or 80% or 75% of the period of therapeutic administration, or over a period defined by the period of implantation. The maximal variation of drug release rate over the main period of therapeutic administration (for example during the period when therapeutic administration is occurring, at least 12 hours following administration, and before drug release falls below a therapeutically effective level) may not be greater than 50% (or 40% or 30% or 20% or 10%) from the mean rate of release during that period.

The plasma concentration of a drug will be related to the rate of release into the blood balanced against the rate of clearing of the drug from the blood (the drug half-life concentration). The aim of sustained drug delivery is to achieve a therapeutic plasma concentration of the drug over a desirable extended period of time. The desired therapeutic plasma concentration will be a concentration within the therapeutic window below which drug is ineffective, and above which the drug is toxic, or alternatively has no additional beneficial effect.

In a typical embodiment of the invention, a therapeutic dose of the drug as measured by plasma concentration (but alternatively as measured by clinical measurements) will be released over a period of time ranging from at least an hour to at least twelve months. Exemplary drugs suitable for sustained release include, for example, anti-pain medications (e.g. morphine analogues), anti-psychotics (e.g. risperidone or olanzapine), anti-inflammatories (e.g. steroids or NSAIDs), cholesterol lowering drugs (e.g., statins), osteoporosis drugs (e.g. biphosphonates), anti-angeogenics (e.g., anti-VEGF) and contraceptives. In the case of certain drugs such as contraceptives and osteoporosis drugs, sustained release may be desirable over a period of more than a year, for example at least 2, 3, 4, or 5 years. Prior formulations (Norplant) have been used to deliver a therapeutic dose of contraceptives over a period of three years. Long term sustained release formulations will generally be formulated for implantation within the body, e.g., subcutaneously.

The amount (weight) of drug released over a period of time will be related to therapeutically effective plasma concentration of the drug, the potency of the drug and drug kinetics including the residence time in the various tissues of interest and the rate of clearing. In use, the rate of release of a drug from the pharmaceutical formulation of the invention may be no greater than, for example 10ng per hour, or alternatively, 50ng 100ng 500ng 1000ng 2500ng or 5000ng or in other embodiments, no more than 10µg, 50µg or 100µg or 500µg or 1000µg per hour during the first 6 (or 12 or 24 or 36) hours following implantation. Of course, the desired rate of release of the drug will depend on its potency, its therapeutic window and its half life. For example, Risperidone may be released over a period of time to provide a serum concentration of about 15 ng/ml up to about 100 ng/ml. A release of about 4 mg/day (or in other studies between 1 and 60 mg/day) is found to provide adequate therapeutic serum levels of Risperidone.

In practice, when the drug is released from the formulation, it is released into the surrounding environment, which may be, for example, the intestinal lumen, gastric fluid, subcutaneous tissue, blood, muscle, the colonic space, a body cavity, or any surrounding tissue and/or into interstitial space. The rate of the release is not always easy to measure in practice, and the invention includes methods of determining the rate of release of a drug from a formulation of the invention in vitro, such as by using standard methods such as those described in the United States Pharmacopoeia. One such method, employs a large volume (usually about a litre) of phosphate buffer, pH 7.2, maintained at 37 ± 0.5°C and stirred at 100 rpm by using a paddle-type dissolution apparatus (such as may be obtained from Electrolab, Mumbai, India). Drug release studies are generally carried out in triplicate and the mean values are calculated. Many standard methods are well known, and when release rates are described and discussed herein, such release rates may be calculated and compared by these standard methods.

In some embodiments, the formulation is such that less than 20%, or less than 15 %, or less than 10% (or in other embodiments, less than 1%, 3%, 5% or 7%) of the total drug loaded into the formulation is released from the formulation *prior to activation* of the formulation by a specified condition which triggers a substantially greater rate of release. Such release triggers include, for example, a change in pH and/or swelling of the polymer by hydration and/or contact with an enzyme and/or heating of the formulation. Such heating can for example make use of one of the means described above. Immediately following the specified condition, the formulation may release drug quickly as a bolus, or steadily over a period of time. For example it may release at least 50% (or 30%, 60% or 75%) of the total loaded drug over a period of not more than 1, 3, 5, 7, 10, 20, 30, 45, 60, 120 or 360 minutes following activation. In embodiments wherein a bolus release is required, substantially all the drug may be released over a very short period of time, such as not more than 1, 2 or 3 minutes, or in other embodiments where longer release is desired, over a period of not more than 30 minutes.

In some embodiments, the rapid melting characteristic of ECC polymers is used to provide formulations which can be administered in the body and transported to a targeted disease site with little or no release of the drug during transport, but which can be triggered to release the drug at the site by targeted radiation which melts the ECC polymer at the site. In this way a large dose of a drug can be delivered specifically at the target site when needed. This is particularly useful when dealing with toxic drugs that are best delivered in a site-specific manner and not systemically. Site-specific delivery also reduces the dose needed. Such drugs include cytotoxic agents such as chemotherapeutics. The external source of radiation may be, for example, a focused beam of radiation, e.g. infra-red (IR) or radiation of a similar wavelength. For example the radiation source may have a wavelength of about 400 -1500 nm, e.g. 500 - 1200 nm or 700 - 1100 nm. This heat-induced release mechanism may be particularly useful in embodiments wherein the formulation is targeted to a specific site prior to release of the drug. Such embodiments include formulations to target tumour tissue whereby, upon heating, a chemotherapeutic agent is released locally, providing a concentrated bolus of drug at the target site, but minimizing systemic exposure.

For example, a chemotherapeutic agent such as carboplatin may be formulated with a ECC polymer and a targeting ligand such as an antibody or folate that binds selectively to a cell surface receptor differentially expressed on the surface of a specific type tumour cell, for example a colon cancer cell. The formulation may for example be formulated as an emulsion and delivered orally. The emulsion will pass through the stomach and gut to the colon where it will preferentially adhere to tumour tissue. Excess formulation will be voided. An infra-red (IR) source will be used to target the tumour site, heating the adhered formulation and releasing the Carboplatin at high concentration to the target site. Little or none of the drug will be released from the formulation until the formulation is heated. This method of administration reduces the amount of drug delivered systemically and thereby reduces the damaging effects of toxic drugs on healthy cells. In certain embodiments, a material which can be heated by radiation, e.g. carbon nanotubes, may be mixed into the formulation. A similar embodiment might employ venous delivery of a liquid or emulsion formulation wherein the drug formulation specifically binds to a target at any location reachable by the circulation.

Initial burst release of a drug is often (but not always) undesirable, and particular embodiments of the present invention provides a reduced burst effect due to the interactions between the drug and the crystalline side chains of the polymeric carrier material. In certain embodiments the formulation of the invention releases a drug so that the initial release of drug does not exceed the upper threshold of the therapeutic window. In other embodiments it exceeds the upper therapeutic window threshold for no more than 1 hour (or alternatively no more than 2, 3, 4, 5, or 6 hours). In certain embodiments the formulation of the invention releases a drug so that the maximum initial release of drug does not exceed by more than 50% the mean rate of release of drug (the mean rate measured over a period from 3-12 hours post administration). Alternatively the maximum initial release of drug does not exceed by more than 20% or 30% or 40% or 70% or 100% the mean rate of release of drug as measured over a period from 6-24 hours or 1-12 hours or 12-48 hours post administration.

Often a formulation of the invention may be implanted directly into a subject to provide acceptable burst effect and sustained release. But for some formulations, and particularly with some particularly toxic drugs that have a narrow upper limit to their therapeutic window, pre-treatment of the formulation may be done to reduce burst effect in vivo. Pre-treatment may simply involve soaking the formulation in a biocompatible liquid or elution buffer, for example phosphate buffered saline (PBS) or water for a period of time before implantation. In such an embodiment, the solid polymer formulation would be removed from its packaging, and placed in the elution buffer for, for example 30 minutes to an hour. In some embodiments longer soaking times may be desirable, for example overnight. Soaking may continue for any duration, for example for up to (or alternatively not more than) 1, 3, 6, 9, 12, 24 or 46 hours.

This period of soaking will allow the formulation to equilibrate with its liquid environment and may provide some degree of hydration (if hydration does occur), such that any drug that has migrated to the surface, or any drug that would provide a burst release when implanted into the subject will be released during the soaking period. After the soaking period the formulation will be implanted into the subject to provide sustained release of the drug within a therapeutic window.

In one specific embodiment the formulation is provided as part of a kit, wherein the kit comprises a two-chamber blister pack having the formulation in a first chamber and a liquid (an elution buffer, e.g. PBS) in a second chamber, and wherein an operator can break the seal between the first and second chambers allowing the liquid to contact the formulation. The formulation is pre-treated in the liquid for a period of time, for example from 30 minutes to 2 hours to allow any burst to dissipate. The formulation is then removed aseptically from the packaging and implanted into the recipient subject, for example implanted subcutaneously by use of a simple trocar.

### Induced Drug Release

Temperature or pH changes may be used to induce drug release at a desired time and/or location. In some embodiments, a small increase in the temperature of the formulation, in situ in an organism, for example from body temperature to a temperature above body temperature, but below a temperature which would damage the organism around the formulation, causes the ECC polymer to melt, so that it changes from a crystalline to an amorphous structure. The combination of the loss of crystallinity, and in the case of a hydrogel, loss in volume, are factors which are useful in the delivery of the drug.

Focused infrared radiation, for example, can be applied from outside the body to selectively heat a target within the body. In some embodiments a formulation may be delivered to a particular location within a patient without appreciable release of drug. When and where desired, an IR beam can be used to heat the target, causing a bolus release of drug at the desired location and time. One such embodiment uses a formulation that includes a ligand that binds specifically to a target. Such ligands may be, for example, antibodies, and are discussed further herein. The formulation is delivered to the patient, for example intravenously; the ligand allows the formulation to bind to and accumulate at a target, such as cancer tissue; a focused IR beam is applied to the target tissue, thereby releasing a bolus of drug.

Another method of promoting the release of a drug associated with a ECC polymer inside a human body or other organism is to supply heat to the ECC polymer, for example through one or more of a hot compress, hot water, hot contact wand, hot pad, infrared radiation, and penetrating heat via near infrared radiation; and/or by generating heat within the formulation. For example, the formulation can include a component which will generate heat when exposed to electromagnetic radiation of a particular range of wavelengths. Such components include for example carbon fiber nanotubules or particles which absorb near-IR light in the range of about 700-1100 nm, silver particles or gold particles.

In various other embodiments a transdermal formulation may be applied to the skin and heat may be produced by chemical or electrical means, or even by physical means such as rubbing with the hand to produce friction-induced heat, to provide a bolus release of drug. Such embodiments are further described herein.

The formulations may include a remotely controlled release mechanism to trigger release of the drug, e.g., an electromechanical mechanism that allows an operator to release drug by use of a radio-frequency signal, sent, for example, over a computer network such as the Internet or a hospital intranet.

Such a mechanism may employ, for example, solenoids, electromagnetic gates or other microfluidic flow control mechanisms. Such mechanisms are well known in the microfluidic and MEMS industry. A remotely operated heating element may be provided to heat the formulation and thus release a bolus of drug.

In some embodiments a therapeutically effective dose of the drug may be released at a predetermined time wherein the drug is released from the formulation by one or more of the following changes in condition (i) heating the formulation, (ii) hydration of the formulation, (iii) exposing of the formulation to an enzyme, or (iv) changing the pH of the environment surrounding the formulation.

### Methods of administration of the formulations and diseases that may be treated using the formulations of the invention

The formulations of the invention may be administered in various ways. Exemplary types of administration include: ingestion, injection, parenteral administration, oral, subdermal, transdermal, transmucosal, intrathecal, intramuscular, inhalation, and application to the skin. Formulations and devices of the invention include lozenges, capsules, tablets, pills, pessaries, lavages, suppositories, inhalable powders, creams, solutions, suspensions, oral suspensions, emulsions, micelle systems, nanoparticles, vesicles, nanocapsules, microcapsules, microparticles, microspheres, microparticles, particles, hydrogels, pills, tablets, including sub-lingual tablets, depots and injectables. Hydrogels are sometimes a preferred form in that they can be mixed as a powder with the drug in a solvent medium to hydrate the hydrogel/drug mixture.

The preferred formulation and route of application will depend upon the drug and the desired application. Sustained release formulations will need to reside in or on the body for the period of desired release, therefore such formulations will be implants, implanted devices and patches, for example transdermal, or transmucosal. Pills, capsules and syrups are typical for oral/gastro-intestinal formulations that are resistant to acid degradation. Because of their susceptibility to acid or severe environments, polypeptides and proteins are often administered by injection as suspensions or solutions. Topical application of other drugs will typically be in suspension or cream form.

Diseases that may be treated by the formulations of this invention include, but are not limited to, cardiac diseases, cardio-vascular diseases, neoplastic diseases, inflammation and all diseases associated with the inflammatory process such as arthritis, auto-immune diseases such as lupus, allergies, infectious diseases (viral, bacterial, fungal, protozoan and prion), endocrine diseases such as diabetes, obesity and all forms of weight-related diseases and disorders whether clinically significant or not, hypertension, psychosis, anxiety, depression or any psychological disease, addiction, erectile dysfunction or any sexual dysfunction, blood-lipid diseases such as high cholesterol, Alzheimer's, alopecia, propecia, mange, incontinence, pain, Meniere's disease, migraine, tinnitus, osteoperosis, fertility problems and any other disease of an organism for which administration of a drug is warranted.

Organisms that can be treated by the present invention include for example human beings and other mammals including bovines, primates, equines, bovines, porcines, canines and felines, and also birds, fish, ungulates and members of the Mustelidae family such as Meles meles.

### Other Medical Applications

Other medical applications of the invention are described below. They are: --
1) Delivery of polynucleotides including RNA interference (RNAI) using cationic ECC polymers.
2) ECC polymers as tissue scaffolds.
3) ECC polymers as ocular inserts
4) ECC polymers in drug eluting stent applications

### 1. Delivery of Polynucleotides Including Rna Interference (RNAi) Using Cationic ECC Polymers

It is known to deliver polynucleotides by complexing cationic polymers such as poly(lysine) with negatively charged polynucleotides. Such cationic polymers usually have amines at the terminal ends of short conformationally flexible side chains. These polymer delivery systems have limitations such as non-biocompatibility/biodegradability or poor gene transfection efficiency.

Cationic ECC polymers can be formulated so that the backbone and/or the terminal groups contain amine groups which are available to interact with polynucleotides or their derivatives to form polynucleotide/polymer complexes for efficient delivery of polynucleotide. Such complexes, and methods of using such complexes to deliver polynucleotides, are novel and form part of the present invention. Delivery systems using cationic ECC polymers may have several potential advantages. First, the polymers can be biocompatible, biodegradable or excretable from the human body. Secondly, the interaction of the polymer with the polynucleotide will stabilize the polynucleotides and prevent the degradation of the polynucleotide. Thirdly, the versatility of ECC polymer chemistry allows one to prepare a neutral or slightly-positively-charged complex that more easily crosses through the hydrophobic membranes (e.g., cytoplasmic, lysosomal, endosomal, nuclear) of the cell, thus increasing transfection efficiency. Finally, the versatility of ECC polymer chemistry enables preparation of a variety of polymers which complex well with polynucleotides and therefore, potentially further improve transfection efficiency.

In one embodiment, the cationic ECC polymer is preferably at least partially protonated to form a complex with the negatively charged polynucleotide.

In another embodiment of the invention, the polymers may have amines at the terminal positions of the backbone chains.

A polynucleotide can be complexed with the ECC polymer by any means known in the art. The complex can be in the form of particles, for example ranging in size from 100 micrometer to 10 nanometers, and can be prepared using known techniques. Preferably the complexes are combined with pharmaceutical excipients to form pharmaceutical formulations suitable for delivery to animals including humans. In one embodiment, the polynucleotide/polymer complexes form nanoparticles that are useful in the delivery of polynucleotides to cells. The polynucleotides used in this application may be any nucleic acid, including but not limited to, RNA, RNA interference (RNAi), DNA, ribozyme, DNA-RNA hybridizer, and oligonucleotides. The polynucleotides may be of any size or sequence, and they may be single- or double-stranded. Polynucleotides may be modified by any means including but not limited to methylation, phosphorylation, end-capping, etc. These derivatives may also be prepared by modification of functional groups of a polynucleotide, including but not limited to, the bases, sugars, and/or phosphate linkages, by techniques known in the art. The polynucleotides may contain any sequence which encodes a protein or a peptide. The polynucleotide may also contain regulatory regions that control the expression of a gene. In a preferred embodiment, the polynucleotide in the invention is an antisense agent or small interfering RNA (siRNA).

### 2. ECC Polymers As Tissue Scaffolds

It is known to use synthetic biomaterials as a matrix for cell or tissue scaffolds for tissue engineering in orthopaedic, soft tissue, cartilage and nerve repair. Typically those scaffolds are prepared by seeding biomaterials with cells in culture. An interaction of biomaterials with cells yields a three-dimensional scaffold/biological composite with functional tissue equivalents. Those scaffolds can then be grafted into the same patient to function as a replacement tissue. Some such systems are useful for organ tissue replacement where there is a limited availability of donor organs or where, in some cases (e.g. young patients) inadequate natural replacements are available. Various materials including biodegradable polyesters--in particular poly(lactide-co-glycolide)s-have been used in the studies.

This invention can make use of an ECC polymer as a scaffold for tissue engineering applications. The ECC polymers are useful for this purpose because they can comprise a macropore structure with a high level of interconnectivity between macropores.

The preferred ECC polymer for use in this application is an ECC-PLGA type block copolymer, for example as described above. The ECC polymer may be in any physical configuration, including but not limited to, bead, disk, particle, film, rod, body shape, etc. A preferred matrix is three dimensional, and is permeable to body fluids.

In one embodiment, the ECC polymer matrix has a macroporous structure including interrupted pore walls and polymer struts, since such a structure allows cell growth which is crucial for the development of three-dimensional tissue. In one aspect of this application, a polymer scaffold comprises macropores in the millimeter size range, preferably, ranging in size between 0.1 mm to 5 mm. In another aspect, the polymer scaffold has a porosity of 50-90%. Preferably, macropores in the polymers are interconnected, and the voids of the macrostructure are substantially continuous.

In one embodiment, the porous polymer matrix changes its size less than 50% after cells are seeded into the matrix, and/or changes in porosity size less than 50% when cells are seeded on the matrix.

In order to promote cell attachment to a polymer matrix, a preferred matrix may contain any moieties that enhance cell adhesion to the matrix. Those moieties include, but are not limited to cell-binding peptides/proteins such as Arg-Gly-Asp (RGD) and Tyr-Ile-Gly-Ser-Arg (YIGSR), etc. The cell-binding compounds may be attached to a polymer chain, or a surface of a preferred matrix by electrostatic forces, covalent bonding, or any other mechanisms using techniques known in the art.

The ECC polymer matrix may have a coating. The coating may be attached to the matrix by electrostatic forces, covalent bonding, or any other mechanism. Coating materials include, but are not limited to, hydrophilic polymers such as PEG, PVE, PEO, amphiphilic polymers such as Pluronics, and biological agents such as bone morphogenetic protein, etc.

The ECC polymer matrix optionally also includes one or more of the following: --
(1) Synthetic or naturally occurring polymers (hydrophobic or hydrophilic); examples include, but are not limited to, PLGA, poly(ethylene glycol), poly(ethylene oxide), polypropylene oxide, polypropylene glycol, poly(vinyl alcohol), polyurethane, collagen, gelatin, chitasan, sugar, and various derivatives of cellulose, etc.
(2) Amino acids, organic or inorganic compounds and salts, including but not limited to polylysine, collagen, gelatin, fibronectin, glycosaminoglycan, hydroxyapatite, sodium carbonate, sodium phosphate, calcium carbonate, and calcium phosphate.
(3) A compound selected from the group consisting of transition metal oxides, transition metal sulfates, transition metal carbonates, transition metal phosphates, and transition metal nitrates.
(4) Pharmaceutically acceptable additives or excipients.
(5) A living cell or a bioactive agent, including but not limited to proteins, peptides, vaccines, DNA, RNA etc.

The invention includes a method of making a porous polymer matrix using a formulation including a solvent, a porogen, and a ECC polymer. Preferably, the porogen is biodegradable or biocompatible or excretable or of natural origin. Typically, the method of making a porous polymer matrix includes (a) combining an ECC polymer solution and a porogen to form a mixture, and (b) extracting the porogen from the mixture and precipitating the polymer substantially simultaneously.

This invention includes a method of making a tissue scaffold using an ECC polymer matrix. Typically, cell seeding of the polymer scaffold is performed using conventional methods well known to those of skill in the art. Cells are seeded onto the polymer scaffold and cultured for three-dimensional growth on polymer scaffolds under suitable growth conditions. The cultures are fed with media appropriate to establish the growth thereof.

The introduced cells attach to the connective tissue within the matrix and are fed by the blood vessels. The elements of these materials can be overlaid with a second material to enhance cell attachment. The polymer matrix is configured to provide access to ingrowing tissues to form adequate sites for attachment of the required number of cells for viability and function and to allow vascularization and diffusion of nutrients to maintain the cells initially implanted. Cells of various types can be grown throughout the present polymer scaffold. In an embodiment, cell types include, but are not limited to, cells selected from the group consisting of hepatocytes, pancreatic islet cells, fibroblasts, chondrocytes, osteoblasts, exocrine cells, cells of intestinal origin, bile duct cells, parathyroid cells, thyroid cells, cells of the adrenal-hypothalamic-pituitary axis, heart muscle cells, kidney epithelial cells, kidney tubular cells, kidney basement membrane cells, nerve cells, blood vessel cells, cells forming bone and cartilage, and smooth and skeletal muscle cells.

In one embodiment, an ECC polymer matrix serves as a support or scaffold on which cell growth occurs in vitro prior to implantation in vivo (pre-seeding). In another preferred embodiment, the scaffolds may also be used directly in vivo, without being pre-seeded with cells.

In an embodiment, ECC polymer scaffolds are suitable for the growth of connective tissues, like bone, cartilage, paradontal tissue, as well as dental tissues and other organs, such as liver or breast tissue.

In one embodiment, an ECC polymer scaffold is used for biomedical applications, such as bone or tissue replacement. The ECC polymer can be biodegradable, or biocompatible but not biodegradable, when a permanent scaffold is needed to support other tissue or in applications where the scaffold is used as a filter.

The scaffold can contain therapeutic agents and act as a delivery vehicle for biologically active moieties, e.g. from growth factors, genes and drugs.

### 3. ECC Polymers As Ocular Inserts

Pharmaceutical formulations comprising ECC polymers can be used in ocular treatments, e.g. in injectable and biodegradable ocular inserts. The formulations can be biologically inert, biodegradable, non-allergenic, and insoluble in tear liquid.

The ECC polymer is preferably insoluble in tear liquid. The ECC polymer may be a mixture of one or more ECC polymers with one or more synthetic or naturally occurring polymers (hydrophobic or hydrophilic). Examples include, but are not limited to, PLGA, poly(ethylene glycol), poly(ethylene oxide), polypropylene oxide, polypropylene glycol, poly(vinyl alcohol), polyurethane, collagen, gelatin, chitosan, sugar, kaolin, talc, magnesium trisilicate, and various derivatives of cellulose, etc.

The ECC polymer can optionally be mixed with one or more of
(1) a Cy-containing compound, including, but not limited to a fatty acid/alcohol containing the same or different Cy moieties;
(2) amino acids, inorganic salts or organic compounds, including but not limited to sodium carbonate, sodium phosphate, calcium carbonate, and calcium phosphate;
(3) pharmaceutically acceptable additives or excipients well known in the art.

The drug may be incorporated in several ways into the ECC polymer.

In one embodiment, an ocular insert and method include the covalent attachment of a drug, to an ECC polymer that contains functional groups, through labile linkages such as an ester linkage. The drug is released over time by hydrolysis of the labile bonds.

In another embodiment of the invention, a drug is dissolved or dispersed in the ECC polymer and the mixture is formed into an appropriately shaped article for ocular delivery.

An ocular insert containing an active substance to be delivered is typically prepared in one of several ways. For example, the polymer can be melted, mixed with the active substance and cast or injection molded into a device. Such melt fabrication requires an ECC polymer having a melting point that is below the temperature at which the drug degrades or is otherwise damaged. An ocular insert containing a drug can be prepared by solvent casting where the ECC polymer is dissolved in a solvent and the drug dissolved or dispersed in the polymer solution and the solvent is then evaporated. Solvent processes require that the ECC polymer be soluble in organic solvents.

Another method is compression molding of a mixed powder of the ECC polymer and the drug or polymer particles loaded with the active agent.

The ocular insert is preferably sized, shaped and adapted for easy insertion and prolonged retention in the eye for administration of a therapeutically effective amount of the substance to be delivered. The insert can be any desired shape, including but not limited to a thin flexible film, a bead, a microparticle (a microsphere, nanosphere, or microcapsule), a rod, and a disc, etc. The insert can be prepared in any desired thickness and width.

The ocular insert may have a coating. The coating may be attached to the matrix by electrostatic forces, covalent bonding, or any other mechanism. Coating materials include, but are not limited to hydrophilic polymers such as PEG, PVA, PEO, amphiphilic polymers such as Pluronics, and any other materials for desirable properties.

One embodiment, the delivery device provides the controlled release of a drug for 1-7 days or longer after a single administration, without the need to remove the device after the compound has been released.

Any drug can be delivered in this way. Examples include, but are not limited to, water soluble or water insoluble drugs such as nonsteroidal anti-inflammatory compounds, anesthetics, chemotherapeutic agents, immunosuppressive agents, steroids, antibiotics, antivirals, antifungals, steroidal antiinflammatories, and anticoagulants.

### 4. ECC Polymers In Drug Eluted Stent Applications

Various families of drug polymer chemistries have been used for coating a stent to increase the effectiveness of stenting procedures and control drug-elution properties. Polymeric coatings of polymers such as a polyamide, parylene or a parylene derivative, poly(butyl methacrylate), poly(ethylene-co-vinyl acetate), PLA-PEO and PLA-PCL have been reported. However, drug-eluting stents using such polymers exhibit deficiencies such as poor biocompatibility, unsatisfactory mechanical properties during roll down and expansion of the stent, and undesirable drug release profiles. A beneficial drug-polymer system needs to be biocompatible, have satisfactory mechanical properties, and provide a desired elution rate for a specific drug. The versatile chemistry of the ECC polymers allows for the selection of desired properties, thus providing versatility in geometric design, strut size and drug delivery options to mitigate the deficiencies of the known procedures.

This invention includes the use of ECC polymers in drug-eluting stent applications as either a drug-polymer coating on a stent or a drug- polymer stent. Thus, one embodiment of this invention is a stent coated by a coating of drug and an ECC polymer. Such a drug eluting stent coated by an ECC polymer is flexible for insertion into a body lumen in an expanded configuration, and is also rigid for support of the body lumen, and achieves a desirable drug release profile.

Another embodiment of this invention is a biodegradable stent for implantation into a body lumen.

The polymeric material used for making a stent or coating a stent is a mixture of one or more ECC polymers.

In various embodiments, the ECC polymer is mixed with one or more of the following
(1) synthetic or naturally occurring polymers (hydrophobic or hydrophilic), for example PLGA, poly(ethylene glycol), poly(ethylene oxide), polypropylene oxide, polypropylene glycol, poly(vinyl alcohol), polyurethane, collagen, gelatin, chitosan, sugar, and various derivatives of cellulose, etc.,
(2) a crystallizable small additive, for example a fatty acid/alcohol, alkylated PEG and the like, optionally containing the same or different Cy moieties,
(3) pharmaceutically acceptable excipients including, but not limited to carbon nanotubes, hydroxyapatite and other biodegradable additives that can improve the mechanical properties of the polymer formulation, and
(4) pharmaceutically acceptable additives or excipients known in the art to assist targeted drug release.

One embodiment of this invention is a system for treating a vascular disease, including a catheter; a stent that is coupled to the catheter; and a polymeric coating of a ECC polymer or polymer blend that is dispersed on the stent framework. A therapeutic agent is in contact with the polymer matrix.

One embodiment of this invention is a method of manufacturing a drug-polymer coated stent, including the steps of forming a polymeric solution containing ECC polymers; applying the polymeric solution onto a stent framework; and drying the polymeric solution.

One embodiment of this invention is a method of treating a vascular condition, including the steps of inserting a drug-polymer coated stent within a vessel of a body, and eluting at least one therapeutic agent from the drug-polymer coated stent into the body. The drug-polymer coated stent comprises an ECC polymer and at least one therapeutic agent in contact with the ECC polymer.

One embodiment of this invention is a method of manufacturing a drug-biodegradable polymeric stent, including the steps of melting an ECC polymer, dispersing therapeutic agents into the polymer melt, and applying the process of casting or injection molding into a device to form a stent.

One embodiment of the invention is a method of treating a vascular condition, including the steps of inserting a drug-biodegradable polymeric stent within a vessel of a body, and eluting at least one therapeutic agent from the drug-polymeric stent into the body. The drug-polymeric stent comprises an ECC polymer and at least one therapeutic agent in contact with the blended matrix.

### Devices

Various devices can make use of the pharmaceutical formulations of the invention. Such devices include coated devices, prosthetic devices, implanted devices, pills, depots and patches. In some embodiments a formulation of the invention is coated onto an implantable medical device. Such implanted devices include stents, pacemakers, catheters, screws, staples, sutures, prosthetic devices etc. Such devices can be made by applying the formulation to an interior or exterior surface of a device by dipping, painting, spraying, misting, rolling, or other method. In some methods, the formulation is heated to above its melting temperature and applied to the device and then dried to form a solid coating.

In another aspect, the devices are implanted depots such as solid, liquid and gelatinous depots that can be deposited into the tissue of a subject, for example by subcutaneous injection. In some embodiments, the formulation is heated to above its melting temperature and injected (or otherwise flowed) into the desired location, e.g. subcutaneously, where it cools and forms a depot which will then release drug in situ. In some such embodiments, Tp is only just above body temperature, e.g. less than 55°C, preferably less than 48°C, so that the formulation may be implanted with minimal discomfort or tissue damage. Once implanted, the formulation cools and crystallizes. Such an application may be of particular use in providing an implanted device that moulds to fit the internal shape of a cavity such as within an ablated area of an infracted heart, within bone tissue or subdermally.

In some embodiments, e.g. some depot embodiments, the formulation includes a non-polymeric substance, e.g. sucrose acetate isobutyrate (SAIB) and non-water soluble liquid materials having a viscosity of at least 5,000 centipoise at 37ºC that do not crystallize under ambient physiological conditions, for example those described in US 6,992,065 (Okumu). Such substances, for example SAIB, which are preferably highly hydrophobic, can enhance drug loading and/or modify drug release profiles. In some embodiments the material is physically mixed with the ECC polymers; in other embodiments it may be used as is or incorporated into the ECC polymer.

In some embodiments, devices include a formulation containing a radio-opaque dye or other marker so that the formulation can be imaged by fluoroscopy or X-ray. Many radio-opaque substances are commonly known and used such as barium, silver and gold.

There are many drugs which can be administered by implanting a specific device into a patient. There are today commercial devices which work by swelling pressure from a polymer which forces a drug to be dispersed from a small cylindrical implanted container. In a similar manner a drug can be administered according to the present invention by implanting an ECC polymer / drug matrix, the ECC polymer optionally formulated with other polymers and/or formulating ingredients known for drug delivery devices. Or, a drug associated with a ECC polymer could be dispensed from a cylindrical metal or plastic tube or device with holes in the device to allow passage of fluid into, and dissolution of drug from, the device. Using implanted devices is particularly of interest for delivery of drugs over a long period of time, for example 1-2 months or longer, e.g. up 6 months, up to a year or in some cases for more than a year. As examples of drugs that might be dispensed are hormones, for example birth control drugs which can be administered like many hormones in very minute quantities, so that a reasonably sized device can be implanted into a patient, for example, in the upper arm, to dispense drug over the long desired time. Particularly if the device has to be explanted, this kind of an implant is only contemplated where long term delivery of small amounts of drug is desirable from a relatively small device which does not cause discomfort to the patient once it is implanted. Another area where this kind of a device would be suitable is in the drug delivery of compliance drugs, for example, anti-psychotic drugs, used by patients who often have difficulty in complying to simple oral pill administration on a regular basis. The implanted device would provide a constant and desirable release of a small amount of anti-psychotic drug as prescribed to assure patient compliance and avoid incidences which are costly to the medical system if the patient does not comply with the dosage schedule of an oral administered pill form. Also, the use of an implanted device may be suitable for patients with chronic or severe pain either from a terminal disease - e.g. cancer - or from a painful and chronic back, hip or other nerve-induced pain issue. Also, the melting behavior of the ECC polymers makes it possible to design an implanted device such that, at body temperature substantially no drug is released but at slightly above body temperature, for example 42° C, a small release of drug will occur. The release can for example be triggered by a radio signal to a very small responder in the device which heats only a portion with of the polymer drug matrix to release a dosage amount of drug. It is also well known that many patients, especially elderly patients, often overdose on medications or take medications on incorrect schedules resulting in emergency hospitalization. There is, therefore, need for a device which will deliver a drug (or in some cases multiple drugs) over a long period, or at triggered intervals over a long period, in the dosage required.

This invention includes a drug delivery device which comprises a pharmaceutical formulation comprising an ECC carrier and a drug, and which has one or more of the following characteristics.
a) It can be implanted into a patient. For convenience, the device would always be implanted at the same location in a patient (in the inside area of an upper arm) so that it could be easily located by, for example, doctors.
b) It is biocompatible.
c) It delivers one or more drugs over a long period, e.g. 3-6 months, or at triggered intervals over a long period, in the dosage required.
d) It is capable of delivering a plurality of drugs
e) it comprises a plurality of segments which are connected to each other, preferably in a way that makes it possible to replace each segment with a similar or different segment. Optionally, some or all of the segments can deliver the same drug, optionally in the same dosage, thus enabling delivery of a desired dosage (e.g. as a multiple of the dosage delivered by each segment, so that a doctor could specify a particular number of segments of a particular drug to raise or lower the dose). Optionally, some or all of the segments can deliver different drugs to treat a number of infirmities simultaneously (e.g. to treat depression, schizophrenia, anemia, high cholesterol or heart disease). Each segment can for example comprise a solid molded section of the drug/ECC polymer, optionally supported by a biologically inert structure which permits delivery of the drug and which connects adjacent segments
f) one or more of the segments could contain a radiopaque additive, so that it would show up on an x-ray scan, and/or an RFID tag.

### Examples EC1 EC10

Examples EC1-EC10 describe the preparation of ECC polymers, and are summarized in Table EC1 below. Table EC1 identifies the starting materials and the amounts thereof in grams, and molecular weight and DSC characteristics of the various products. In the PLGA polymers used in the Examples, the molar ratio of (units derived from) GA to LA was 1:1. After the Table, there is a detailed account of the procedures used in each of Examples EC1-EC10. The heading of each detailed example includes an abbreviation indicating the polymer produced in the example, in the form "PLGA", indicating that the polymer is an unmodified PLGA [in comparative Example EC1(C)], or "nECC-PLGA", where n is 1, 2, 3, 4 or 6, indicating that the polymer is an end-capped PLGA containing 1, 2, 3, 4 or 6 Cy moieties.

**TABLE EC1**

| Ex. #. | EC1 | EC1(C) | EC2 | EC3 | EC4 | EC5 | EC6(a) | EC6(b) |
|---|---|---|---|---|---|---|---|---|
| # | 341-1-3 | 341-1-0 | 338-80 | 341-41-5 R | 338-55 | 338-56 | 338-75 | 338-77 |
| GA | 51.925 | 57.011 | - | 55.53 | 55.53 | - | - | |
| LA | 70.291 | 77.175 | - | 75.18 | - | - | - | |
| C6 PLGA | | | | | 5.034 | - | - | |
| C22 A | 11.15 | - | - | - | - | - | - | |
| C22 OH | C22 OH | - | - | 5.53 | 1.145 | 2.071 | - | 1.272 |
| EC1 | - | - | 16.055 | - | - | 5.033 | - | |
| SUCAN | - | - | 2.002 | - | - | - | - | |
| EC2 | - | - | - | - | - | - | 6.73 | |
| Glycerol | - | - | - | - | - | - | 60 mL | |
| EC6(a) | - | - | - | - | - | - | | 1.27 |
| To | | | 50.55 Tg16.2 | | 45.95 | 54.77 | | 52.11 |
| Tp | 58 | Tg 18.7 | 57.06 Tg19.2 | 59.8 | 54.17 | 59.89 | | 58.46 |
| Mn | 812 | 1165 | 633 | 665 | 4986 | 3912 | | 6091 |
| Mw | 981 | 1484 | 879 | 1184 | 7080 | 7119 | | 14,235 |

| Ex. #. | EC7 (a) | EC7 (b) | EC8 | EC9 (a) | EC9 (b) | EC9 (c) | EC10 (a) | EC10 (b) |
|---|---|---|---|---|---|---|---|---|
| ID # | 338-91 | 338-93 | 338-92 | 338-85 | 338-87 | 338-88 | 338-83 | 338-84 |
| C22 acid | | 2.044 | | | | 2.504 | | 1.583 |
| SUCAN | | | | 1.465 | | | | |
| EC1 (C) | | | | 15.027 | | | | |
| EC2 | | | | | | | 7.049 | |
| Glycerol | 35 mL | | 0.282 | | 25 mL | | 30 mL | |
| EC3 | 15.077 | | 8.133 | | | | | |
| EC7(a) | | 3.16 | | | | | | |
| EC9(a) | | | | | 9.85 | | | |
| EC9 (b) | | | | | | 2.2 | | |
| EC10 (a) | | | | | | | | 1.86 |
| To | | 56.69 Tg 9,96 | 25.37 (1) 44.48(2) | | | 51.34 | | 51.54 |
| Tp | | 60.35 Tg 18.97 | 30.72(1) 49.29(2) | | | 57.16 | | 57.15 |
| Mn | | 4774 | 2479 | | | 45,370 | | 6893 |
| Mw | | 18,385 | 3373 | | | 160,900 | | 15,545 |

### Example EC1 - preparation of 1 ECC-PLGA (ID #341-1-3)

The GA, LA and C22OH were placed in a 1000 mL vessel equipped with a mechanical stirrer and heated to 140ºC. After stirring for 15 to 25 min, vacuum was applied to remove water. The reaction was continued under reduced pressure at 140ºC for at least 16hrs. A clear pale yellow viscous liquid was removed from the vessel while it was still hot. After cooling to room temperature, 85 gram of opaque solid (341-1-3) was obtained.

### Example EC1(C) -- preparation of PLGA (ID #341-1-0)

The GA and LA were reacted following the procedure of Example EC1. A clear solid, 84 g, (341-1-0) was obtained.

### Example EC2- preparation of 1 ECC-PLGA (ID #338-80)

The ECC-PLGA (ID # 341-1-3) and SUCAN (molar ratio of 1:1), and a catalytic amount of PTSA were combined with 200 mL toluene in a 250 mL round bottom flask fitted with a condenser and Dean-Stark trap. The mixture was stirred under reflux with a magnetic stir bar under nitrogen in a 135°C oil bath overnight. The temperature was increased to 150°C and stirring continued for 2 days, after which toluene was removed by distillation over 4.5 hours. Reaction progress was monitored by FTIR through loss of anhydride peaks at 1864 cm-1 and 1782 cm-1, loss of broad PLGA alcohol peak from 3600-3400 cm-1, and increase in broad acid OH peak from 3300-2400 cm-1. The product was purified by first dissolving in 50 mL warm DCM and adding 50 mL water. The layers were separated in a separatory funnel and the water layer was extracted 1 x with 25 mL DCM. The organic layers were combined and washed 3x with 25 mL water. The DCM layer was dried over anhydrous magnesium sulfate which was removed by gravity filtration. The resulting filtrate was condensed under reduced pressure to give 16.3 g of product (338-80).

### Example EC 3 - preparation of 1 ECC-PLGA (ID #341-41-5R)

The GA (55.53g), LA (75.18g) and C22 acid (5.53g) were reacted following the procedure of Example EC1. An opaque solid, 90 g, (341-41-5R) was obtained.

### Example EC4 - preparation of 2ECC-PLGA (ID #338-55)

The PLGA (polymer having Mn 3127 obtained from Durect, Pelham, AL) and the C22 acid (molar ratio 1:2) and a catalytic amount of PTSA were combined in a 100 mL round bottom flask with 30 mL toluene. The mixture was stirred with a magnetic stir bar under nitrogen and heated for 1 hour until all solids dissolved and the toluene was refluxing. Toluene was removed under reduced pressure over 4 hours followed by continued heating under nitrogen at 140°C for 3.5 days until completely reacted. Reaction progress was monitored by FTIR through loss of the 1708 cm-1 carboxylic acid carbonyl peak and conversion to an ester carbonyl peak at about 1757 cm-1 (overlap with PLGA ester carbonyl at the same shift), and loss of the broad acid OHpeak from 3300-2400 cm-1. The product was purified by first dissolving in 35 mL of warm DCM followed by cooling for 5-10 minutes on dry ice until visibly cloudy with a white precipitate. Gravity filtration over fluted filter paper was performed to remove a white solid. Solvent was removed from the solution under reduced pressure yielding 4.3 grams of product (338-55).

### Example EC5 - preparation of 2ECC-PLGA (ID #338-56)

The polymer ID #341-1-3 and the C22 acid (molar ratio 1:1) and a catalytic amount of PTSA were placed in a 250 mL round bottom flask with 50 mL toluene. The mixture was stirred with a magnetic stir bar under nitrogen in a 140°C oil bath for a quarter hour until all solids dissolved. Toluene was removed under reduced pressure over 2.5 hours followed by continued heating under nitrogen at 140°C for 3.5 days until completely reacted. Reaction progress was monitored by FTIR as in Example EC4. Any residual toluene was removed under reduced pressure for 1 hour. The product was purified by first dissolving in 40 mL of warm DCM followed by cooling for 5-10 minutes on dry ice until cloudy with white precipitate. Gravity filtration over fluted filter paper was performed to remove a white solid. Solvent was removed from the solution under reduced pressure yielding 3.5 grams of product (338-56).

### Example EC6 - preparation of 3ECC-PLGA (ID #338-77)

(a) The polymer ID #338-80 and the glycerol were combined in a 250 mL round bottom flask. The reaction was stirred with a magnetic stir bar under nitrogen in a 130°C oil bath overnight, until reacted. Reaction progress was monitored by taking small samples of the reaction mixture and extracting with DCM, and monitoring by FTIR through the loss of the broad acid OH peak between 3200-2400 cm-1 and the appearance of the glycerol alcohol peak from 3600-3100 cm-1. The product was purified by addition of 100 mL DCM and 100 mL water, stirred and shaken vigorously, and added to a separatory funnel. The resulting layers were separated, and the water layer extracted 3x with 50 mL DCM. The organic layers were combined and washed 3x with 20 mL water, and then dried over anhydrous magnesium sulfate, which was subsequently removed by gravity filtration through fluted filter paper. This extraction and washing process was repeated on the water layers a second time to remove any remaining product, with all resulting organic layers combined and solvent removed under reduced pressure to yield 1.27 grams of intermediate product (ID #338-75).
(b) The intermediate product ID #338-75 and the C22 acid, and a catalytic amount of PTSA and 200 mL toluene when mixed in a 250 mL round bottom flask fitted with a Dean Stark trap. The mixture was stirred under reflux with a magnetic stir bar under nitrogen in a 130°C oil bath overnight. The temperature was then increased to 135°C and stirred under reflux for 24 hours until complete. Reaction progress was monitored by the loss of the broad glycerol OH peak from 3600-3100 cm-1, the disappearance of the broad acid OH stretch from 3200-2400 cm-1, and the loss of the C22 acid carbonyl peak at 1706 cm-1 and conversion to ester carbonyl peak at about 1754 cm-1 (overlap with PLGA ester carbonyl peaks). The product was purified by dissolving in warm DCM and cooled over dry ice for 5-10 minutes until a white precipitate was formed, which was removed by gravity filtration through fluted filter paper. The filtrate was condensed under reduced pressure to produce 1.93 g of product (ID #338-77).

### Example EC7 - preparation of 3ECC-PLGA (ID #338-93

(a) The polymer ID #341-41-R, the glycerol (large molar excess) and a catalytic amount of PTSA were combined in a 100 mL round bottom flask. The mixture was stirred by magnetic stir bar under nitrogen and heated in a 130°C oil bath overnight until reaction was complete. Reaction progress was monitored by taking small samples of reaction mixture and performing a mini-extraction with DCM, with progress monitored through FTIR through the loss of broad acid OH peak from 3300-2300 cm-1 and appearance of broad glycerol OH peak from 3700-3100 cm-1 in product. For purification 50 mL water and 20 mL DCM was added to the reaction flask, warmed, and stirred vigorously until all was dissolved. Using a separatory funnel, the organic layer was isolated and the water layer extracted 2x with 30 mL DCM. The organic layers were then combined and washed 3x with 20 mL water, then dried over anhydrous magnesium sulfate which was then removed by gravity filtration through fluted filter paper. Solvent was removed under reduced pressure to give 3.42 g reaction intermediate (ID #338-91).
(b) The reaction intermediate ID #338-91 and the C22 acid (molar ratio about 1:2.4), 65 mL toluene, and a catalytic amount of PTSA were combined in a 100 mL round bottom flask fitted with condenser and Dean-Stark trap. The mixture was stirred by magnetic stir bar under nitrogen and placed in a 135°C oil bath, and allowed to reflux for 4.5 days until reaction was complete and yielded approximately 4 grams of product (ID #338-93). Reaction progress was monitored by FTIR through the loss of broad glycerol OH peak from 3700-3100 cm-1, loss of broad C22 acid OH peak from 2800-2300 cm-1, and loss of C22 acid carbonyl peak at 1707 cm-1 and strengthening of 1750 cm-1 ester peak due to acid conversion to ester bond.

### Example EC8 - preparation of 3ECC-PLGA (ID #338-92)

The polymer ID #341-41-5R and the glycerol (molar ratio about 2.25:1) and a catalytic amount of PTSA were combined in a 3 neck 250mL round bottom flask. The reaction was stirred by magnetic stir bar and heated under nitrogen in a 135°C oil bath. The reaction was stirred for 1.5 days until reaction was complete and removed from heat to produce the final product (ID #338-92). Reaction progress was monitored by FTIR through the loss of the broad acid OH peak especially from 2800-2400 cm-1, the loss of the glycerol OH peak from 3700-3100 cm-1, and the slight shift of acid carbonyl peak from 1749 cm-1 to 1753 cm-1 due to ester formation (overlap with PLGA ester carbonyl peaks).

### Example EC9 -preparation of 4ECC-PLGA (ID #338-88

(a) The polymer ID #341-1-0 and the SUCAN (molar ratio 1:1.1) and 150 mL toluene were mixed in a 250 mL round bottom flask fitted with a condenser and Dean-Stark trap. The mixture was stirred by magnetic stir bar under nitrogen in a 135°C oil bath while refluxing. The mixture was stirred for 3 days until fully reacted. Reaction progress was monitored by FTIR through the loss of SUCAN peaks at 1864 cm-1 and 1782 cm-1 and loss of PLGA OH peaks from 3700-3300 cm-1. For purification about 50 mL warm DCM and about 50 mL water were added to the reaction flask and stirred vigorously. The layers were then separated by separatory funnel, with the water layer extracted 2 times each with 30 mL with DCM. The DCM layers were combined and then washed with about 30 mL of water once only. The resulting organic layers were dried over anhydrous magnesium sulfate that was subsequently removed by gravity filtration through fluted filter paper. Solvent was removed under reduced pressure to yield 15.08 g of Intermediate 1 (ID #338-85).
(b) The intermediate ID #338-85 and the glycerol (large excess) were combined in a 250 mL round bottom flask. The mixture was stirred by magnetic stir bar under nitrogen in a 130°C oil bath for 20 hours until complete. Reaction progress was monitored by FTIR through loss of the broad acid OH peak between 3200-2500 cm-1 and the appearance of the glycerol alcohol peak from 3700-3200 cm-1. The reaction was purified by adding ∼ 50 mL warm DCM and ∼50 mL water to the flask and stirring vigorously. The layers were then separated in a separatory funnel, and the water layer extracted 2 times each with 50 mL DCM. All organic layers were combined and washed 3 times each with ∼40 mL of water. The organic layer was then dried over anhydrous magnesium sulfate, which was then removed from gravity filtration through fluted filter paper. Solvent was removed under reduced pressure to give 2.41 g of Intermediate 2 (ID #338-87).
(c) The intermediate ID #338-87 and the C22 acid (molar ratio 1:4.5) and a catalytic amount of PTSA were combined in a 250 mL round bottom flask with 150 mL toluene. The mixture was stirred with a magnetic stir bar under nitrogen and stirred in a 135°C oil bath under reflux, while fitted with a condenser and Dean-Stark trap. The mixture was stirred overnight, at which point solvent was drained and the flask placed under reduced pressure to remove the remaining toluene. The reaction was then stirred in the 135°C oil bath under nitrogen for 24 hours until complete and removed from heat. The reaction progress was monitored by FTIR through the loss of the broad glycerol OH peak from 3700-3200 cm-1, the loss of the broad acid OH stretch from 3100-2400 cm-1, and the loss of the C22 acid carbonyl peak at 1703 cm-1 and conversion to ester carbonyl peak at about 1757 cm-1 (overlap with PLGA ester carbonyl peaks). The product was purified by dissolving the sample in warm DCM to dissolve and cooled over dry ice for 5-10 minutes until cloudy with white precipitate. Precipitate was removed by gravity filtration through fluted filter paper and the filtrate was condensed under reduced pressure to produce the final product (ID #338-88) with yield of 2.91 g.

### Example EC10 -preparation of 3ECC-PLGA (ID #338-84)

The procedure of Example EC6 was followed, but using the product from Example EC2 (338-80, 7.049 g) and 30 mL of glycerol to obtain intermediate 338-83. The intermediate 338-83 (1.86 g) was reacted with C22 acid (1.583 g) as in Example EC6 until the reaction was complete. After purification, 2.84 g of product (338-84) was obtained.

### Preparation and Testing of Pharmaceutical Formulations containing ECC polymers and comparative polymers.

Mixtures of drug (either risperidone or diclofenac sodium) and polymer were prepared at 9.1 % drug loading by mixing the drug with the polymer in TCM at the ratio of drug/polymer/TCM=1/10/35 (wt/wt/wt) at 70°C, followed by evaporation of TCM in a 70°C oven, and removal of residual TCM under reduced pressure at 70ºC. The drug/polymer mixtures formed a single phase above the Tp of the polymer.

Release samples were prepared as a thin flat disc in a 20mL scintillation vial (28 x 61 mm = OD x H) by loading 0.5 gram of the prepared polymer/drug mixture into the vial and warming to 65-80°C, allowing the mixture to flow and fuse together. After cooling the mixture to a temperature below Tp, a solid disc with a smooth uniform surface formed at the bottom of the vial.

### Release test for risperidone samples

The risperidone samples were tested by covering the polymer/drug disc with 12.6 grams of a buffer solution at pH=5.5(50mM ionic strength, 150 mM NaCl and containing 0.01 % w/v Tween-20). The buffer solution was removed and replaced by 12.6 grams of fresh buffer solution at scheduled sampling times, e.g. at 1 min, 2hr, 6hr, daily, weekly, or as necessary. The amount of risperidone released into the sample solution was measured by UV-Vis against a standard curve established by using the absorption signal at λ = 276.93nm. The buffer solution used to test the risperidone/polymer samples was prepared by adding to 100 mL DI water, 0.16 g of monosodium phosphate monohydrate and 1.04 g disodium phosphate, followed by NaCl and Tween-20 to a final concentration of 0.9% and 0.01% w/v, respectively. The pH was adjusted to 5.5 with 0.1 N HCl as necessary.

### Release test for diclofenac sodium samples

The diclofenac sodium samples were tested by covering the polymer/drug with 5 grams of buffer solution with pH=7.4 (50mM ionic strength, 150 mM NaCl and containing 0.01% w/v Tween-20). The buffer solution was removed and replaced by 5 grams of fresh buffer solution at scheduled sampling times, e.g. at 1 min, 2hr, 6hr, daily, weekly, or as necessary. The amount of diclofenac sodium released into the sample solution was measured by UV-Vis against a standard curve established by using the absorption signal at λ = 276.93nm. The buffer solution used to test the diclofenac sodium samples was the same as that used to test the risperidone polymer samples, except that its pH was adjusted to 7.4 with 0.1 N HCl or 0.1 N NaOH as necessary.

### Figures 1-8

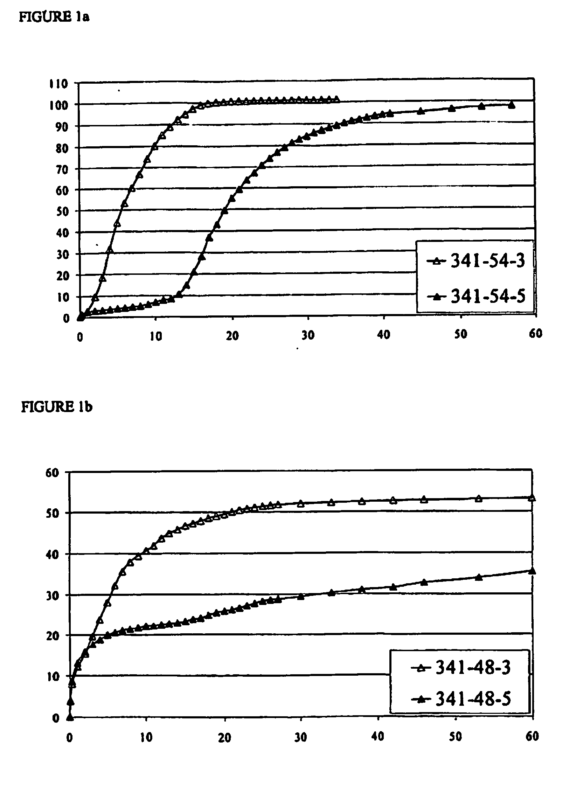

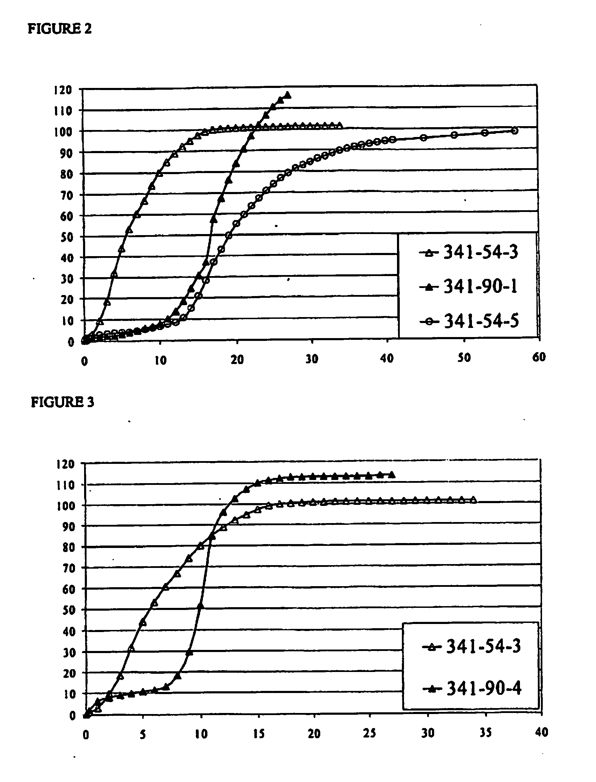

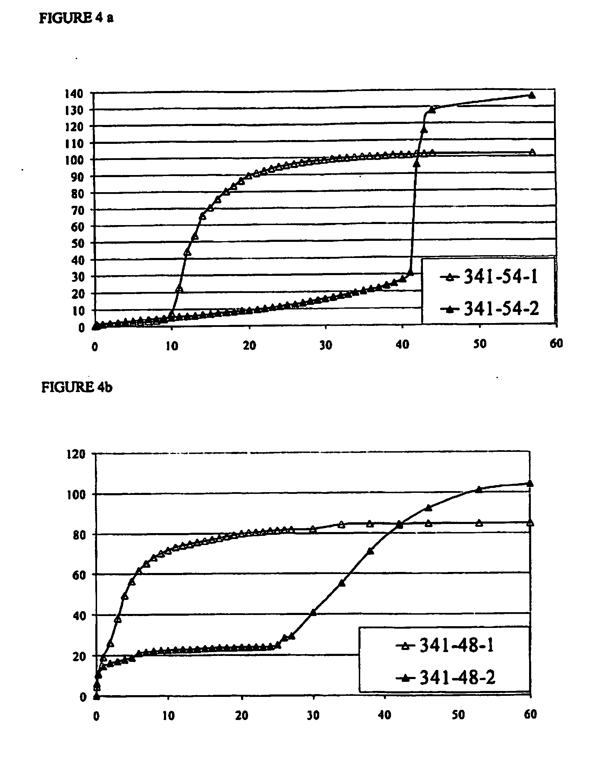

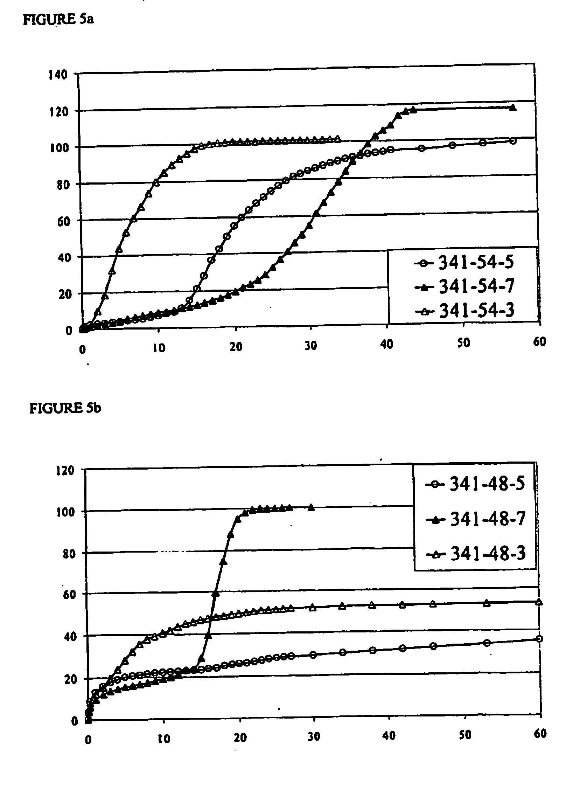

The results of the testing are shown in Figures 1-8, in which the vertical axis shows the total percentage release ("cumulative release") of the drug, and the horizontal axis shows the time in days. Thus, the Figures show the cumulative release of
(i) in Figure 1 a, risperidone from test sample ID #341-54-3 based on the unmodified PLGA polymer ID #341-1-0 prepared in Example EC1 (C); and test sample ID #341-54-5, based on polymer ID #341-1-3 prepared in Example EC 1;
(ii) in Figure 1 b, diclofenac sodium from test sample ID #341-48-3 based on a commercially available unmodified PLGA, and from test sample 341-48-5 based on polymer ID#341-1-3 prepared in Example EC1;
(iii) in Figure 2 risperidone from test sample ID #341-54-3 (above), from test sample ID #341-90-1, based on polymer ID #338-80 prepared in Example EC3, and from test sample #341-54-5 based on polymer ID #341-1-3 prepared in Example EC1;
(iv) in Figure 3, risperidone from test sample #341-54-3 (above) and from test sample 341-90-4 based on polymer ID number 341-41-5R prepared in Example EC 3;
(v) in Figure 4a, risperidone from test sample ID #341-54-1 based on a commercially available unmodified PLGA, and from test sample ID #341-54-2, based on polymer ID number 338-55 prepared in Example EC 4;
(vi) in Figure 4b, diclofenac sodium from test sample ID #341-48-1 (above) and from test sample ID #341-48-2 based on polymer ID #338-55, prepared in Example EC 4;
(vii) in Figure 5a, risperidone from test sample #341-54-5 based on polymer ID #341-1-3 prepared in Example EC1, from test sample ID #341-54-7 based on polymer ID #338-56 prepared in Example EC5, and from test sample ID #341-54-3 (above);
(viii) in Figure 5b, diclofenac sodium from test sample #341-48-5 based on polymer ID #341-1-3 prepared in Example EC1, from test sample #341-48-7, based on polymer ID #338-56 prepared in Example EC5, and from test sample ID #341-48-3 based on polymer ID #341-1-0 prepared in Example EC1 (C);

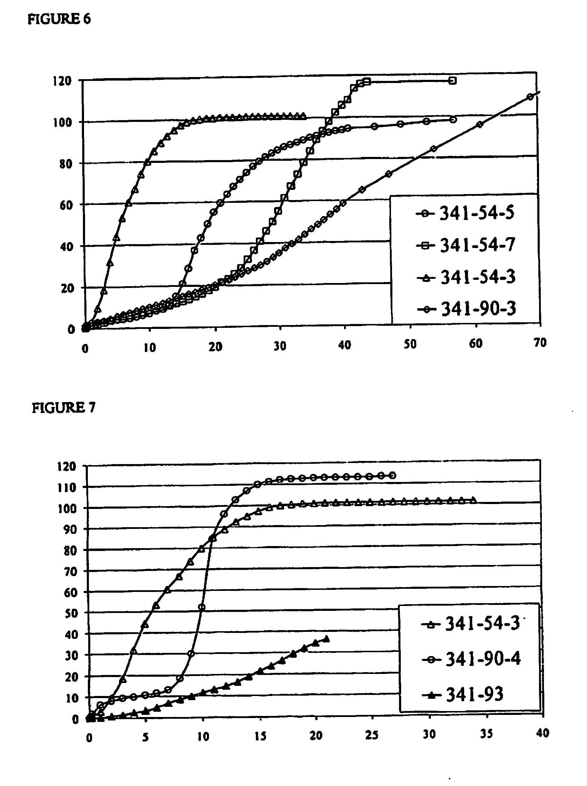

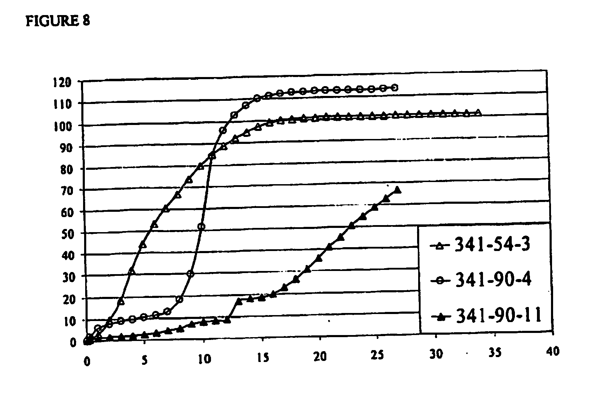

(ix) in Figure 6, risperidone from test sample ID #341-54-5 based on polymer ID #341-1-3 prepared in Example EC1, from test sample ID #341-54-7 based on polymer ID #338-56 prepared in Example EC5, from test sample ID #341-54-3 based on polymer ID #341-1-0, prepared in Example EC1 (C), and from test sample #341-90-3 based on polymer ID #341-1-3 prepared in Example EC1; (x) in Figure 7, risperidone from sample ID #341-54-3 based on polymer ID #341-1-0 prepared in Example EC1(C), from sample ID #341-90-4 based on polymer ID #341-41-5R prepared in Example EC3, and from test sample ID #341-93 based on polymer ID #338-93 prepared in Example EC7; and (x) in Figure 8, risperidone from test sample ID #341-54-3 based on polymer ID #341-1-0, prepared in Example EC1, from test sample ID #341-90-4 based on polymer ID #341-41-5 R prepared in Example EC3, and from test sample ID #341-90-11 based on polymer ID #338-92, prepared in Example EC8.

## Claims

1. A pharmaceutical formulation which comprises an ECC polymer and a drug associated with the ECC polymer, the ECC polymer
(A) comprising a plurality of polymeric molecules each of which consists essentially of
(i) a polymer backbone which comprises a plurality of repeating units having the formula
-CF¹F²-CO-O- (1)
wherein
F¹ is hydrogen and F² is hydrogen or methyl, the repeating units being the same or different, and
(ii) at least one terminal unit which has the formula
-b- Cy (2)
wherein
Cy is an n-alkyl moiety containing 18-24 carbon atoms, and
b is a bond or a moiety which has a valence of at least 2, which links the Cy moiety to the polymer backbone, and which optionally contains one or more additional Cy moieties;
(B) having a crystalline melting temperature, Tp, of at least 40°C, an onset of melting temperature, To, such that the value of (Tp -To) is less than Tp^{0.7}, and a heat of fusion, ΔH, of at least 5 J/g, Tp, To and ΔH being measured on a differential scanning calorimeter (DSC) as hereinbefore described; and
(C) having a number average molecular weight, Mn, measured by gel permeation chromatography with a light scattering detection method,
of less than 10,000.

2. A formulation according to claim 1 wherein said at least one terminal unit comprises moieties having the formula
-Qx(-b-Cy)_{q} (Q2)
wherein Qx is a moiety having a valence of at least (q+1) and q is at least 2.

3. A formulation according to claim 1 wherein at least some of the polymeric molecules contain at least three Cy moieties, and the polymer has a heat of fusion of at least 10 J/g.

4. A formulation according to claim 1 wherein at least one of the Cy moieties includes a polyoxyalkylene moiety.

5. A formulation according to claim 1 wherein the ECC polymer
(A) consists essentially of a plurality of polymeric molecules each of which consists essentially of
(i) a polymer backbone which consists essentially of a plurality of repeating units having the formula
―CH₂―CO―O― (1A)
and a plurality of repeating units having the formula
-CH(CH₃)-CO-O- (1B)
and
(ii) a plurality of terminal units each of which has the formula
-b- Cy (2)
wherein
Cy is an n-alkyl moiety containing 18-24 carbon atoms, and
b is a bond or a moiety which has a valence of at least 2, which links the Cy moiety to the polymer backbone and which optionally contains additional Cy moieties;
(B) has a crystalline melting temperature, Tp, of at least 40°C, an onset of melting temperature, To, such that the value of (Tp -To) is less than 10°C, and a heat of fusion of at least 5 J/g, Tp, To and the heat of fusion being measured on a differential scanning calorimeter (DSC) as hereinbefore described; and
(C) has a number average molecular weight, Mn, measured by gel permeation chromatography with a light scattering detection method, of less than 8,000.

6. A formulation according to claim 5 wherein the polymer has a molecular weight less than 5000.

7. A formulation according to claim 5 wherein the polymer contains 10-40% by weight of Cy moieties.

8. A formulation according to claim 5 wherein at least one of the terminal units has a formula selected from the group consisting of
(2A) -Cy
(2B) -CO-O-Cy
and
(2C) -R_{pbalc}-CO-Cy Cy
where R_{pbalc} is the residue of a polyol.

9. A formulation according to claim 5 wherein at least one of the terminal units has a formula selected from the group consisting of
-CH₂-CH(OH) -CH₂-O-CO- Cy
and
-CH₂-CH(O-CO-Cy)-CH₂-O-CO-Cy.

10. A formulation according to claim 1 wherein the ECC polymer has been prepared by a process which comprises copolymerizing (a) lactic acid and glycolic acid and their cyclic dimers, lactide and glycolide, which results in the repeating units of formula (1) and (b) one or more monomers or components which will result in the terminal units of formula (2) or which can be converted into terminal units of formula (2).

11. A formulation according to claim 1 wherein the ECC polymer has been prepared by a process which comprises copolymerizing one or more of lactic acid, glycolic acid, and an alcohol containing a Cy moiety.

12. A formulation according to claim 1 wherein the ECC polymer has been prepared by a process which comprises (1) copolymerizing lactic acid, glycolic acid and a polyol to form a PLGA glyceride ester, and (2) end capping the PLGA glyceride ester with an excess of a carboxylic acid containing a Cy moiety.

13. A method of making a pharmaceutical formulation according to any one of the preceding claims which comprises mixing a drug with an ECC polymer as defined in any one of the preceding claims, the drug having a maximum temperature to which it can be exposed without damage, and the drug being mixed with the polymer at a temperature at which the polymer is liquid and which is below the maximum temperature.

14. A method according to claim 13 which is carried out in the absence of any liquid other than the polymer.

15. A pharmaceutical formulation according to any one of claims 1-12 for use in therapy or diagnosis.

## Patentansprüche

1. Pharmazeutische Formulierung, die ein ECC-Polymer und ein mit dem ECC-Polymer assoziiertes Arzneimittel umfasst, wobei das ECC-Polymer
(A) eine Mehrzahl von polymeren Molekülen umfasst, die jeweils im wesentlichen aus
(i) einer Polymerhauptkette umfassend eine Mehrzahl von Struktureinheiten mit der Formel
-CF¹F²-CO-O- (1)
worin
F¹ Wasserstoff ist und F² Wasserstoff oder Methyl ist, wobei die Struktureinheiten gleich oder verschieden sind, und
(ii) mindestens einer endständigen Einheit mit der Formel
-b-Cy- (2)
worin
Cy eine n-Alkylgruppe mit 18-24 Kohlenstoffatomen ist und
b eine Bindung oder eine Gruppe ist, die eine Wertigkeit von mindestens 2 aufweist und die Cy-Gruppe mit der Polymerhauptkette verbindet, und welche gegebenenfalls eine oder mehrere zusätzliche Cy-Gruppen enthält;
bestehen,
(B) eine kristalline Schmelztemperatur, Tp, von mindestens 40°C, eine Onset-Schmelztemperatur, To, so dass der Wert von (Tp-To) weniger als Tp^{0,7} ist, und eine Schmelzwärme, ΔH, von mindestens 5 J/g, aufweist, wobei Tp, To und ΔH auf einem Differentialscanningkalorimeter (DSK) wie vorstehend beschrieben gemessen werden, und
(C) ein Zahlenmittel des Molekulargewichts, Mn, gemessen durch Gelpermeationschromatographie mit einem Lichtstreuungsdetektionsverfahren, von weniger als 10.000 aufweist.

2. Formulierung nach Anspruch 1, worin die mindestens eine endständige Einheit Einheiten mit der Formel
-Qx(-b-Cy)_{q} (Q2)
umfasst, worin Qx eine Gruppe mit einer Wertigkeit von mindestens (q +1) ist und q mindestens 2 ist.

3. Formulierung nach Anspruch 1, worin mindestens einige der polymeren Moleküle mindestens drei Cy-Gruppen enthalten und das Polymer eine Schmelzwärme von mindestens 10 J/g aufweist.

4. Formulierung nach Anspruch 1, wobei mindestens eine der Gruppen Cy eine Polyoxyalkylengruppe beinhaltet.

5. Formulierung nach Anspruch 1, wobei das ECC-Polymer
(A) im wesentlichen aus einer Mehrzahl von polymeren Molekülen besteht, die jeweils im wesentlichen aus
(i) einer Polymerhauptkette im wesentlichen bestehend aus einer Mehrzahl von Struktureinheiten mit der Formel
-CH₂-CO-O- (1A)
und einer Mehrzahl von Struktureinheiten mit der Formel
-CH(CH₃)-CO-O- (1B)
und
(ii) einer Mehrzahl von endständigen Einheiten, die jeweils die Formel
-b-Cy- (2)
aufweisen, worin
Cy eine n-Alkylgruppe mit 18-24 Kohlenstoffatomen ist und
b eine Bindung oder eine Gruppe ist, die eine Wertigkeit von mindestens 2 aufweist und die Cy-Gruppe mit der Polymerhauptkette verbindet, und welche gegebenenfalls zusätzliche Cy-Gruppen enthält;
bestehen,
(B) eine kristalline Schmelztemperatur, Tp, von mindestens 40°C, eine Onset-Schmelztemperatur, To, so dass der Wert von (Tp-To) weniger als 10°C ist, und eine Schmelzwärme von mindestens 5 J/g, aufweist, wobei Tp, To und die Schmelzwärme auf einem Differentialscanningkalorimeter (DSK) wie vorstehend beschrieben gemessen werden, und
(C) ein Zahlenmittel des Molekulargewichts, Mn, gemessen durch Gelpermeationschromatographie mit einem Lichtstreuungsdetektionsverfahren, von weniger als 8.000 aufweist.

6. Formulierung nach Anspruch 5, worin das Polymer ein Molekulargewicht von weniger als 5.000 aufweist.

7. Formulierung nach Anspruch 5, worin das Polymer 10-40 Gew.-% Cy-Gruppen enthält.

8. Formulierung nach Anspruch 5, worin mindestens eine der endständigen Einheiten eine Formel ausgewählt aus der Gruppe bestehend aus
(2A) -Cy
(2B) - CO-O-Cy
und
(2C) -R_{pbalc}-CO-Cy
aufweist, wobei R_{pbalc} der Rest eines Polyols ist.

9. Formulierung nach Anspruch 5, worin mindestens eine der endständigen Einheiten eine Formel ausgewählt aus der Gruppe bestehend aus
-CH₂-CH(OH)-CH₂-O-CO-Cy und
-CH₂-CH(O-CO-Cy)-CH₂-O-CO-Cy
aufweist.

10. Formulierung nach Anspruch 1, wobei das ECC-Polymer hergestellt worden ist durch ein Verfahren, das umfasst das Copolymerisieren von (a) Milchsäure und Glycolsäure und ihren cyclischen Dimeren, Lactid und Glycolid, woraus sich die Struktureinheiten der Formel (1) ergeben, und (b) einem/einer oder mehreren Monomeren oder Komponenten, die die endständigen Einheiten der Formel (2) ergeben oder die in die endständigen Einheiten der Formel (2) umgewandelt werden können.

11. Formulierung nach Anspruch 1, wobei das ECC-Polymer hergestellt worden ist durch ein Verfahren, das umfasst das Copolymerisieren von einem oder mehreren von Milchsäure, Glycolsäure und einem Alkohol enthaltend eine Cy-Gruppe.

12. Formulierung nach Anspruch 1, wobei das ECC-Polymer hergestellt worden ist durch ein Verfahren, das umfasst (1) das Copolymerisieren von Milchsäure, Glycolsäure und einem Polyol, um einen PLGA-Glyceridester zu bilden, und (2) die Endgruppenbildung am PLGA-Glyceridester mit einem Überschuss von einer Carbonsäure enthaltend eine Cy-Gruppe.

13. Verfahren zur Herstellung einer pharmazeutischen Formulierung nach irgendeinem der vorhergehenden Ansprüche, umfassend das Mischen eines Arzneimittels mit einem ECC-Polymer wie in irgendeinem der vorhergehenden Ansprüche definiert, wobei das Arzneimittel eine maximale Temperatur aufweist, der es ohne Schädigung ausgesetzt werden kann, und das Arzneimittel mit dem Polymer bei einer Temperatur gemischt wird, bei der das Polymer flüssig ist und die unterhalb der maximalen Temperatur liegt.

14. Verfahren nach Anspruch 13, das in Abwesenheit von irgendeiner Flüssigkeit, die von dem Polymer verschieden ist, durchgeführt wird.

15. Pharmazeutische Formulierung nach irgendeinem der Ansprüche 1 bis 12 zur Verwendung in der Therapie oder Diagnose.

## Revendications

1. Formulation pharmaceutique qui comprend un polymère ECC et un médicament associé au polymère ECC, le polymère ECC
(A) comprenant une pluralité de molécules polymériques dont chacune est constituée essentiellement
(i) d'un squelette polymère qui comprend une pluralité d'unités répétitives répondant à la formule
-CF¹F²-CO-O- (1)
où
F¹ est un atome d'hydrogène et F² est un atome d'hydrogène ou un groupe méthyle, les unités répétitives étant identiques ou différentes, et
(ii) d'au moins une unité terminale qui répond à la formule
-b-Cy (2)
où
Cy est un fragment n-alkyle renfermant 18 à 24 atomes de carbone, et b est une liaison ou un fragment dont la valence vaut au moins 2, qui relie le fragment Cy au squelette polymère, et qui contient facultativement un ou plusieurs fragments Cy supplémentaires ;
(B) ayant une température de fusion des cristallites, Tp, d'au moins 40°C, une température de début de fusion, To, telle que la valeur de (Tp-To) est inférieure à Tp^{0,7}, et une chaleur de fusion, ΔH, d'au moins 5 J/g, Tp, To et ΔH étant mesurées sur un calorimètre à balayage différentiel (DSC) comme décrit ci-dessus ; et
(C) ayant une masse moléculaire moyenne en nombre, Mn, mesurée par chromatographie d'exclusion diffusion avec un procédé de détection de diffusion de la lumière, inférieure à 10000.

2. Formulation selon la revendication 1 dans laquelle ladite au moins une unité terminale comprend des fragments répondant à la formule
-Qx(-b-Cy)_{q} (Q2)
où Qx est un fragment dont la valence vaut au moins (q+1) et q vaut au moins 2.

3. Formulation selon la revendication 1 dans laquelle au moins une partie des molécules polymériques contiennent au moins trois fragments Cy, et le polymère a une chaleur de fusion d'au moins 10 J/g.

4. Formulation selon la revendication 1 dans laquelle au moins l'un des fragments Cy comporte un fragment polyoxyalkylène.

5. Formulation selon la revendication 1 dans laquelle le polymère ECC
(A) est constitué essentiellement d'une pluralité de molécules polymériques dont chacune est constituée essentiellement
(i) d'un squelette polymère qui est constitué essentiellement d'une pluralité d'unités répétitives répondant à la formule
-CH₂-CO-O- (1A)
et d'une pluralité d'unités répétitives répondant à la formule
-CH(CH₃)-CO-O- (1B)
et
(ii) d'une pluralité d'unités terminales dont chacune a la formule
-b-Cy (2)
où
Cy est un fragment n-alkyle renfermant 18 à 24 atomes de carbone, et b est une liaison ou un fragment dont la valence vaut au moins 2, qui relie le fragment Cy au squelette polymère et qui contient facultativement des fragments Cy supplémentaires ;
(B) a une température de fusion des cristallites, Tp, d'au moins 40°C, une température de début de fusion, To, telle que la valeur de (Tp-To) est inférieure à 10°C, et une chaleur de fusion d'au moins 5 J/g, Tp, To et la chaleur de fusion étant mesurées sur un calorimètre à balayage différentiel (DSC) comme décrit ci-dessus ; et
(C) a une masse moléculaire moyenne en nombre, Mn, mesurée par chromatographie d'exclusion diffusion avec un procédé de détection de diffusion de la lumière, inférieure à 8000.

6. Formulation selon la revendication 5 dans laquelle le polymère a une masse moléculaire inférieure à 5000.

7. Formulation selon la revendication 5 dans laquelle le polymère contient 10 à 40% en masse de fragments Cy.

8. Formulation selon la revendication 5 dans laquelle au moins l'une des unités terminales a une formule choisie à partir du groupe constitué
(2A) de -Cy
(2B) de -CO-O-Cy
et
(2C) de -R_{pbalc}-CO-Cy
où R_{pbalc} est le résidu d'un polyol.

9. Formulation selon la revendication 5 dans laquelle au moins l'une des unités terminales a une formule choisie à partir du groupe constitué
de -CH₂-CH(OH)-CH₂-O-CO-Cy
et de -CH₂-CH(O-CO-Cy)-CH₂-O-CO-Cy.

10. Formulation selon la revendication 1 dans laquelle le polymère ECC a été préparé par un processus qui comprend une copolymérisation (a) d'acide lactique et d'acide glycolique et de leurs dimères cycliques, de lactide et de glycolide, qui conduit aux unités répétitives de formule (1) et (b) d'un ou de plusieurs monomères ou composants qui conduira aux unités terminales de formule (2) ou qui peut être convertie en unités terminales de formule (2).

11. Formulation selon la revendication 1 dans laquelle le polymère ECC a été préparé par un processus qui comprend une copolymérisation d'un ou de plusieurs acides parmi l'acide lactique et l'acide glycolique, et d'un alcool contenant un fragment Cy.

12. Formulation selon la revendication 1 dans laquelle le polymère ECC a été préparé par un processus qui comprend (1) la copolymérisation d'acide lactique, d'acide glycolique et d'un polyol afin de former un ester de glycéride PLGA, et (2) la réalisation d'une protection terminale de l'ester de glycéride PLGA à l'aide d'un excès d'acide carboxylique contenant un fragment Cy.

13. Procédé de fabrication d'une formulation pharmaceutique selon l'une quelconque des revendications précédentes qui comprend le fait de mélanger un médicament avec un polymère ECC tel que défini dans l'une quelconque des revendications précédentes, le médicament ayant une température maximale à laquelle il peut être exposé sans dommages, et le médicament étant mélangé avec le polymère à une température à laquelle le polymère est liquide et qui est inférieure à la température maximale.

14. Procédé selon la revendication 13 qui est effectué en absence d'un liquide quelconque autre que le polymère.

15. Formulation pharmaceutique selon l'une quelconque des revendications 1 à 12 à utiliser dans le cadre d'une thérapie ou d'un diagnostic.
